# EUROPEAN PATENT APPLICATION

(11) **EP 4 773 524 A2**
(43) Date of publication of application: **08.07.2026**
(21) Application number: 26179186.7
(22) Date of filing: 24.04.2024
(51) Int. Cl.: H04B 13/00

(54) **CONDUCTIVE COMMUNICATION VECTOR SELECTION BASED ON PHYSICAL AND/OR PHYSIOLOGIC STATE OF PATIENT**

(30) Priority: 17.05.2023 US 202363502893 P; 19.04.2024 US 202418640735; 19.04.2024 US 202418640751
(62) Divisional of application: 25197020.8
(71) Applicant: Pacesetter, Inc., Sylmar, CA 91342 (US)
(72) Inventor: SPROWLS, Mark J, Sylmar, CA 91342 (US); FUJIAN, Qu, Sylmar, CA 91342 (US); WENWEN, Li, Sylmar, CA 91342 (US); BOOTH, Daniel F, Sylmar, CA 91342 (US); BORNZIN, Alexander R, Sylmar, CA 91342 (US); DAWOUD, Fady, Sylmar, CA 91342 (US)
(74) Representative: Barker Brettell Sweden AB

(57) **Abstract**

Devices (106, 109) and methods for improving conductive communication are described herein. One of the devices (106, 109) involved in the conductive communication can be an external device while the other device is an IMD, or both of the devices can be IMDs. In certain embodiments, conductive communication is performed using a first timing scheme that specifies when conductive communication pulses are transmitted relative to one or more cardiac events. A quality metric of the conductive communication is monitored, when the first timing scheme is being used to perform the conductive communication. In response to detecting the quality metric being below a corresponding threshold, when the first timing scheme is being used to perform the conductive communication, the invention changes to using a second timing scheme to perform the conductive communication.

## Description

### FIELD OF TECHNOLOGY

Embodiments of the present technology described herein generally relate to techniques to improve conductive communication between a first device and a second device, wherein at least one of the first and second devices is an implantable medical device (IMD). Embodiments of the present technology also relate to systems and devices that are configured to implement such techniques.

### BACKGROUND

From time to time a non-implanted device needs to communicate with an implantable medical device (IMD), such as a leadless pacemaker (LP), so that the non-implanted device can, for example, program the IMD, interrogate the IMD, and/or obtain notifications and/or other types of diagnostic information from the IMD. Such a non-implanted device, which can also be referred to as an external device, can be, e.g., an external programmer or a remote monitor.

Communication between an external device and one or more IMDs (e.g., LPs) may be facilitated by conductive communication via patient tissue, whereby skin electrodes (that are part of or coupled to the external device) are attached to skin of a patient within which (i.e., in whom) one or more IMDs is/are implanted, and the skin electrodes are used to transmit information to and/or receive information from the IMD(s) via conduction through body tissue of the patient. In other words, the two skin electrodes can be used by the external device to transmit conductive communication signals via patient tissue to individual IMDs. Additionally, or alternatively, the two electrodes can be used by the external device to receive conductive communication signals from individual IMDs. The communication signals transmitted from an external device to an IMD, or vice versa, to achieve conductive communication can be referred to herein as conductive communication signals. The skin electrodes are examples of external electrodes, i.e., non-implanted electrodes. Where conductive communication signals are transmitted from an external programmer to an IMD, the conductive communication signals can also be referred to more specifically as conductive programmer-to-implant (p2i) communication signals, or more succinctly as conductive p2i signals. Where the conductive communication signals are transmitted from an IMD to an external programmer, the conductive communication signals can also be referred to more specifically as conductive implant-to-programmer (i2p) communication signals, or more succinctly as conductive i2p signals. Conductive communication signals are also referred to sometimes as conducted communication signals, and these terms are often used interchangeably. Conductive communication, which relies on the transmission of voltage or current pulses using electrodes of one device through patient tissue, and the reception of the voltage or current pulses using electrodes of another device, is also sometimes referred to as conductive communication or galvanic communication.

An important consideration when using conductive communication is that the orientation of and/or distance between the IMD(s) may change over a cardiac cycle and/or a respiratory cycle, which may cause fading that can adversely affect conductive i2i, p2i and i2p communication. Explained another way, an important consideration for conductive communication is the relative orientation of the devices involved, since the electrical potential between the electrodes used to receive conductive communication signals will depend on their orientation relative to the electrodes used to transmit the conductive communication signals. Suboptimal relative orientation of the electrodes of the devices involved in conductive communication may result in unsuccessful conductive communication and/or energy intensive conductive communication, both of which are undesirable. For example, the locations of the skin electrodes used by an external device, which define a communication vector for the external device, may not provide for good communication signal quality between the external device and an IMD. More generally, the orientation and location of an IMD and the locations of the external electrodes can affect the communication quality between an external device and an IMD. These problems may be exacerbated when there is a need or desire for the external device to communicate with multiple (i.e., two or more) IMDs. For example, it may be the case that a communication vector associated with a pair of skin electrodes attached to a patient's skin provides for good conductive communication signal quality with only one of multiple IMDs. To overcome this problem, the pair of skin electrodes attached to the patient's skin at first locations can first be used to provide for conductive communication between the external device and a first IMD. The skin electrodes can then be moved to second locations and then used to provide for conductive communication between the external device and a second IMD. If the patient also includes a third IMD, the skin electrodes can then be moved to third locations and then used to provide for conductive communication between the external device and the third IMD. Even if a patient only includes a single IMD, it still may be necessary to move one or more of the skin electrodes one or more times before acceptable conductive communication signal quality is achieved between the external device and the single IMD. This repositioning or moving of the skin electrodes can be time consuming for both the patient and the medical personnel, as well as costly in terms of increasing the patient's medical bills.

Additionally, where a patient has multiple IMDs that need to perform implant-to-implant (i2i) communication with one another using conductive communication, unsuccessful or delayed i2i communication may compromise patient monitoring and/or therapeutic efficacy. Where one or more of the IMDs is/are intracardiac leadless pacemakers (LPs) that each have only one pair of electrodes, relative orientations and/or positions of the LPs may vary depending on physiologic variables such as phase of cardiac cycle, posture, breathing patterns, activity, time from implant date, etc., which can affect the quality of the conductive i2i communication between LPs.

Further, where a patient has an extravascular IMD, such as a subcutaneous non-vascular implantable cardioverter defibrillator (NV-ICD) that has or is coupled to more than two electrodes, and is intended to perform conductive i2i communication with one or more LPs, the orientation and/or position of each of the LP(s) relative to the electrodes of the NV-IMD may vary depending on physiologic and/or physical variables, which can affect the quality of the conductive i2i communication between the extravascular IMD and the LP(s), and may compromise patient monitoring and/or therapeutic efficacy. Examples of physiologic variable includes phases of cardiac cycles, posture, respiratory patterns, patient activity, time from implant date, etc. Examples of physical variables include impedance between various communication vectors and non-physiologic electrical potentials between receiver electrodes resulting from post-shock afterpotentials, just to name a few. Changes in the relative orientations of and/or distances between multiple IMDs can alternatively, or additionally, be due to atrial and/or ventricular remodeling resulting from cardiac resynchronization therapy (CRT), progression of heart failure (HF) and/or atrial fibrillation (AF), and/or migration of one or more IMDs.

### SUMMARY

The present invention is defined in the independent claims. Further embodiments of the invention are defined in the dependent claims.

Certain embodiments of the present technology relate to methods for use by a first device that is configured to communicate with a second device using conductive communication, wherein the first device includes and/or is communicatively coupled to at least three electrodes and can perform the conductive communication using a plurality of different conductive communication vectors, wherein each of the plurality of different conductive communication vectors comprises a different combination of the at least three electrodes, wherein the second device includes and/or is communicatively coupled to at least two additional electrodes that can be used to perform the conductive communication, and wherein at least one of the first or second devices comprises an IMD implanted in a patient. In accordance with certain embodiments, such a method for use by the first device includes obtaining information indicative of at least one of a physical or physiologic state of the patient, and identifying which one of the plurality of different conductive communication vectors is a preferred conductive communication vector for communicating with the second device, based on the information indicative of the at least one of the physical or physiologic state of the patient. The method further includes the first device performing the conductive communication with the second device using the preferred conductive communication vector.

In accordance with certain embodiments of the present technology, obtaining information indicative of the at least one of the physical or physiologic state of the patient comprises obtaining information indicative of at least one of a posture or activity level of the patient, and identifying which one of the plurality of different conductive communication vectors is the preferred conductive communication vector for communicating with the second device is performed based the information indicative of the at least one of the posture or activity level of the patient.

In accordance with certain embodiments of the present technology, the information indicative of the at least one of the posture or activity level of the patient comprises, or is based on, one or more signals output from at least one of an accelerometer or a temperature sensor.

In accordance with certain embodiments of the present technology, the information indicative of the at least one of the physical or physiologic state of the patient comprises, or is based on, at least one of an impedance measurement or an electrocardiogram (ECG) or electrogram (EGM) measurement. In accordance with certain embodiments, information indicative of the relative orientation of the devices involved in conductive communication can be based on at least one of an ECG or EGM measurement and/or i2i communication pulse polarity (of received pulses) and/or amplitude of one or more portions (e.g., phases) of a conductive communication signal.

In accordance with certain embodiments of the present technology, the first device comprises an external device, the at least three electrodes (that the first device includes and/or is communicatively coupled to) comprise at least three skin electrodes, and the second device comprises the IMD. In accordance with other embodiments of the present technology, the first device comprises an NV-ICD, and the second device comprises an intracardiac IMD (e.g., an LP), an ICM, or a pulmonary artery pressure monitoring device, but is not limited thereto.

In accordance with certain embodiments of the present technology, the first device is also configured to communicate with a third device that includes and/or is communicatively coupled to at least two further electrodes that can be used to perform further conductive communication, wherein the third device comprises a further IMD implanted in the patient. In certain such embodiments, the method for use by the first device further comprises identifying which one of the plurality of different conductive communication vectors is a further preferred conductive communication vector for communicating with the third device, based on the information indicative of the at least one of the physical or physiologic state of the patient, and performing the further conductive communication with the third device using the further preferred conductive communication vector.

In accordance with certain embodiments of the present technology, the preferred conductive communication vector is used for both transmitting conductive communication signals to the second device and receiving conductive communication signals from the second device.

In accordance with other embodiments of the present technology, the preferred conductive communication vector is used for one of transmitting conductive communication signals to the second device or receiving conductive communication signals from the second device, and a further preferred conductive communication vector is used for the other one of transmitting conductive communication signals to the second device or receiving conductive communication signals from the second device. In certain such embodiments, the method for use by the first device further comprises identifying the further preferred conductive communication vector, and performing the conductive communication with the second device also using the further preferred conductive communication vector.

In accordance with certain embodiments of the present technology, the obtaining, the identifying and the performing are repeated from time to time such that the preferred conductive communication vector may change in response to the at least one of the physical or physiologic state of the patient changing.

In accordance with certain embodiments of the present technology, the at least one of the physical or physiologic state of the patient comprises at least one state of at least one cyclical physiologic cycle that includes at least one of a cardiac cycle, a respiratory cycle, or another type of cyclical physiologic cycle. In certain such embodiments, the obtaining, the identifying and the performing are performed at least twice during a same cyclical physiologic cycle (e.g., during a same respiratory cycle) such that the preferred conductive communication vector changes within the same cyclical physiologic cycle, resulting in at least two of the plurality of different conductive communication vectors being used to perform the conductive communication with the second device during the same cyclical physiologic cycle (e.g., during the same respiratory cycle). Alternatively, or additionally, the obtaining, the identifying and the performing are performed over multiple cyclical physiologic cycles (e.g., over multiple cardiac cycles and/or over multiple respiratory cycles).

Certain embodiments of the present technology can be used to avoid any uncomfortable muscle stimulation that could potentially result for transmission of conductive communication signals by an NV-ICD, or potentially another type of IMD. More specifically, testing can be performed during an implant procedure and/or thereafter to determine a conductive communication threshold voltage that results in patient discomfort due to muscle stimulation, and information indicative thereof can be stored in a memory of an IMD. The IMD can then purposely avoid transmitting conductive communication signals above or near (e.g., within a specified tolerance of) the conductive communication threshold voltage that resulted in patient discomfort due to muscle stimulation. In certain such embodiments, an IMD can switch to using another vector for transmitting conductive communication signals when conductive communication is unsuccessful or has an unacceptable quality at or near the conductive communication threshold voltage, in lieu of continuing to increase an output voltage using that vector. Alternatively, or additionally, the IMD can switch to using another vector for transmitting conductive communication signals if an evoked response of skeletal muscle is detected by a device, e.g., from a sensed electromyography (EMG).

Certain embodiments of the present technology are directed to a device (aka a first device) that is configured to communicate with a second device using conductive communication, wherein at least one of the device or the second device comprises an IMD configured to be implanted in a patient, wherein the device includes conductive communication circuitry, switches between the conductive communication circuitry and at least three electrodes that are part of or communicatively coupled to the device, and a controller. The controller is configured to control the switches to thereby enable the device to perform the conductive communication using a plurality of different conductive communication vectors, wherein each of the plurality of different conductive communication vectors comprises a different combination of the at least three electrodes. The controller is further configured to obtain information indicative of at least one of a physical or physiologic state of the patient. The controller is also configured to identify which one of the plurality of different conductive communication vectors is a preferred conductive communication vector for communicating with the second device, based on the information indicative of the at least one of the physical or physiologic state of the patient, and control the switches to cause the conductive communication with the second device to be performed using the preferred conductive communication vector.

In accordance with certain embodiments of the present technology, the information indicative of the at least one of the physical or physiologic state of the patient comprises information indicative of at least one of a posture or activity level of the patient. In certain such embodiments, the controller is configured to identify which one of the plurality of different conductive communication vectors is the preferred conductive communication vector for communicating with the second device, based the information indicative of the at least one of the posture or activity level of the patient.

In accordance with certain embodiments of the present technology, the device includes at least one of an accelerometer or temperature sensor, and the information indicative of at least one of posture or activity level of the patient comprises, or is based on, one or more signals output from at least one of the accelerometer or temperature sensor.

In accordance with certain embodiments of the present technology, the information indicative of at least one of posture or activity level of the patient comprises, or is based on, at least one of an impedance measurement, or an ECG or EGM measurement.

In accordance with certain embodiments of the present technology, the device comprises an external device, the at least three electrodes (that the device includes and/or is communicatively coupled to) comprise at least three skin electrodes, and the second device comprises the IMD. In accordance with other embodiments of the present technology, the device comprises an NV-ICD and the second device comprises an intracardiac IMD (e.g., an LP). Where the device comprises an NV-ICD, one of the electrodes used by the NV-ICD for conductive communication can be a case electrode (aka a can or canister electrode) and other electrodes used by the NV-ICD for conductive communication can be electrodes located on one or more leads connected to the NV-ICD.

In accordance with certain embodiments of the present technology, the device is also configured to communicate with a third device that includes and/or is communicatively coupled to at least two further electrodes that can be used to perform further conductive communication, wherein the third device comprises a further IMD configured to be implanted in a patient. In certain such embodiments, the controller is further configured to identify which one of the plurality of different conductive communication vectors is a further preferred conductive communication vector for communicating with the third device, based on the information indicative of the at least one of the physical or physiologic state of the patient, and control the switches to cause the further conductive communication with the third device to be performed using the further preferred conductive communication vector.

In accordance with certain embodiments of the present technology, the conductive communication circuitry comprises a conductive communication transmitter and a conductive communication receiver, and the preferred conductive communication vector is used by the conductive communication transmitter for transmitting conductive communication signals to the second device, as well as being used by the conductive communication receiver for receiving conductive communication signals from the second device. In accordance with other embodiments of the present technology, the preferred conductive communication vector is used for one of transmitting conductive communication signals to the second device or receiving conductive communication signals from the second device, and a further preferred conductive communication vector is used for the other one of transmitting conductive communication signals to the second device or receiving conductive communication signals from the second device. In other words, the preferred conductive communication vector used for transmitting conductive communication signals can differ from the preferred conductive communication vector that is used for receiving conductive communication signals. In certain such embodiments, the controller is further configured to identify the further preferred conductive communication vector, and control the switches to cause conductive communication with the second device also using the further preferred conductive communication vector.

In accordance with certain embodiments of the present technology, the controller is configured to identify a new preferred conductive communication vector when the at least one of the physical or physiologic state of the patient changes, and control the switches to cause the conductive communication with the second device to be performed using the new preferred conductive communication vector.

In accordance with certain embodiments of the present technology, the at least one of the physical or physiologic state of the patient comprises at least one state of at least one cyclical physiologic cycle that includes at least one of a cardiac cycle, a respiratory cycle, or another type of cyclical physiologic cycle. In certain such embodiments, the controller is configured to identify the preferred conductive communication vector at least twice during a same cyclical physiologic cycle such that the preferred conductive communication vector changes within the same cyclical physiologic cycle, and control the switches to cause the conductive communication with the second device to be performed using the least two of the plurality of different conductive communication vectors during the same cyclical physiologic cycle.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1 illustrates a system that includes a plurality of implantable medical devices that are implanted in a patent and an external device that can be used to program and/otherwise communicate with the implantable devices.
FIG. 2 is a high level block diagram of an example LP.
FIG. 3 illustrates an example form factor for an LP.
FIG. 4 depicts a sample configuration involving an external programmer and two endocardially implanted LPs.
FIG. 5 depicts a sample configuration involving an external device and two LPs implanted epicardially (on the external heart surface).
FIG. 6 depicts an example of an external device communicatively coupled to three electrodes that are in contact with the chest of a patient.
FIG. 7 is a high level block diagram illustrating example details of an external device that is configured to communicate with one or more IMDs implanted within a patient using conductive communication, wherein the external device includes and/or is communicatively coupled to at least three external electrodes that are in contact with the patient.
FIG. 8 is a high level block diagram illustrating example details of a non-vascular implantable cardioverter defibrillator (NV-ICD) that is configured to communicate with one or more other IMDs implanted within a patient using conductive communication, and configured to communicate with an external device using conductive communication and/or RF communication.
FIG. 9 is a high level flow diagram used to summarize methods for identifying and using one of a plurality of different possible conductive communication vectors, in accordance with certain embodiments of the present technology, wherein the identifying is based on information indicative of a patient's physical and/or physiologic state.
FIG. 10 is a high level flow diagram used to describe methods for testing various different combinations of conductive communication vectors and physical and/or physiologic states of a patient, in accordance with certain embodiments of the present technology, so that a respective preferred conductive communication vectors can be identified for each of a plurality of different physical and/or physiologic states of the patient.
FIG. 11 is a high level flow diagram used to summarize methods of changing between different timing schemes used for conductive communication, in accordance with certain embodiments of the present technology.
FIG. 12 is a high level flow diagram used to summarize methods of selecting or producing a valid bitstream from at least three separate bitstreams produced using at least the separate conductive communication vectors that are used to receive a conductive communication message from a transmitting device.
FIG. 13 illustrates how a composite bitstream can be determined from at least three separate bitstreams by identifying common bits in at least a majority of the at least three separate bitstreams.
FIG. 14 is a high level flow diagram that is used to summarize certain methods for optimizing selecting of a transmission parameter set that is to be used by a first device for transmitting conductive communication messages to a second device, and optimizing receiving conductive communication vectors to be used by the second device for receiving conductive communication messages from the first device.
FIGS. 15A and 15B show an example of how the position of an LP, relative to a case electrode of an NV-ICD and electrodes located on a lead that is connected to the NV-ICD, can change depending upon the posture of the patient in which the LP and NV-ICD are implanted.

### DETAILED DESCRIPTION

Embodiments of the present technology can be used to improve conductive communication between an external device and one or more implantable medical devices (IMDs) in time and cost efficient manners. Embodiments of the present technology can also be used to improve conductive communication between multiple IMDs, e.g., between multiple LPs and/or between an NV-ICD and one or mor LPs, but not limited thereto.

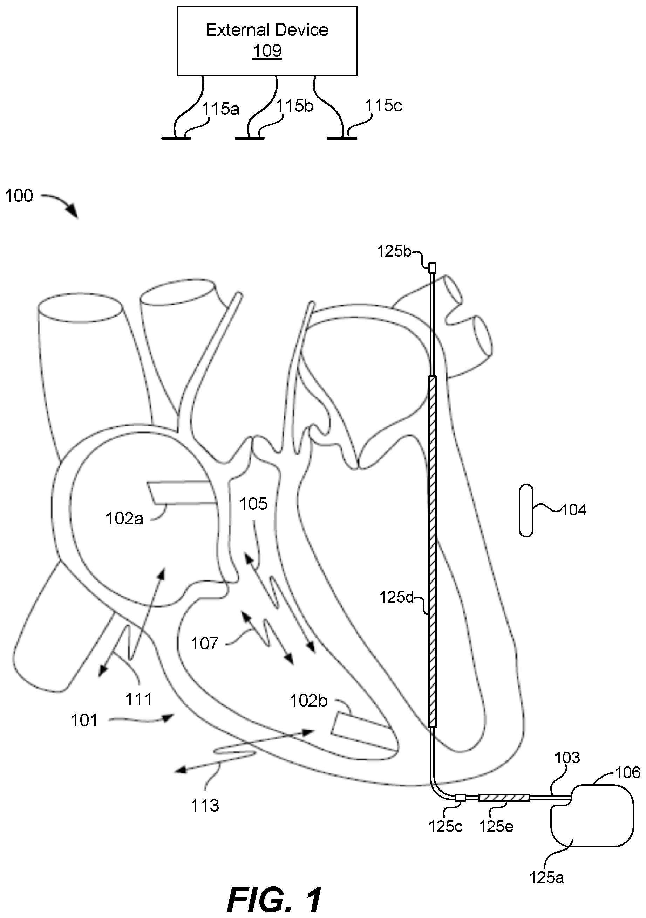

Certain such embodiments improve and preferably optimize the conductive communication signal quality between an external device and one or more IMDs. Other embodiments improve and preferably optimize the conductive communication signal quality between multiple IMDs. However, before providing additional details of the specific embodiments of the present technology, an example environment in which embodiments of the present technology can be useful will first be described with reference to FIGS. 1-3. More specifically, FIGS. 1-3 will be used to describe an example cardiac pacing system, wherein pacing and sensing operations can be performed by multiple IMDs. Such IMDs may include, for example, one or more leadless cardiac pacemakers, an implantable cardioverter defibrillator (ICD), such as a non-vascular ICD (NV-ICD), an insertable cardiac monitor (ICM), and/or a pulmonary artery pressure monitoring device, one or more of which can communicate with an external device (e.g., external programmer) to reliably and safely coordinate pacing and/or sensing operations. A leadless cardiac pacemaker can also be referred to more succinctly herein as a leadless pacemaker (LP). Where the system includes an ICD, the system is also capable of performing defibrillation. Where the only IMD is an ICM or a pulmonary artery pressure monitoring device, the system may only be capable of performing monitoring without performing any therapy.

A conductive communication pulse, as the term is used herein, refers to a voltage pulse that is generated by a device (e.g., by a pulse generator thereof) and emitted between at least a pair of electrodes that corresponds to a conductive communication vector, such that the conductive communication pulse is conducted through patient tissue and may be sensed by at least another pair of electrodes of or electrically coupled to a second device. A conductive communication message, as the term is used herein, can include a sequence of such conductive communication pulses that collectively impart information, e.g., after being decoded and/or demodulated. A conductive communication signal, as the term is used herein, can include one or more conductive communication pulses that collectively make up one or more conductive communication messages, or collectively make up at least a portion of at least one conductive communication message, i.e., a conductive communication signal includes one or more conductive communication pulses.

FIG. 1 illustrates a system 100 that is configured to be at least partially implanted in a heart 101. The system 100 includes LPs 102a and 102b located in different chambers of the heart 101. The LP 102a is located in a right atrium, while LP 102b is located in a right ventricle. The LPs 102a and 102b can communicate with one another to inform one another of various local physiologic activities, such as local intrinsic events, local paced events, and/or the like. The LPs 102a and 102b may be constructed in a similar manner, but operate differently based upon which chamber LP 102a or 102b is located. The LPs 102a and 102b may sometimes be referred to collectively herein as the LPs 102, or individually as an LP 102.

In certain embodiments, the LPs 102a and 102b communicate with one another, and/or with an ICM 104, and/or with an ICD 106, by conductive communication through the same electrodes that are used for sensing and/or delivery of pacing therapy. The LPs 102a and 102b also use conductive communication to communicate with a non-implanted external device 109 (e.g., an external programmer) having two or more electrodes (e.g., 115a, 115b and 115c) placed on the skin of a patient within which the LPs 102a and 102b are implanted. While not shown (and not preferred, since it would increase the size and power consumption of the LPs 102a and 102b), the LPs 102a and 102b can potentially include an antenna and/or telemetry coil that would enable them to communicate with one another, the ICD 106 and/or a non-implanted device using RF and/or inductive communication. While only two LPs 102 are shown in FIG. 1, it is possible that more than two LPs can be implanted in a patient. For example, to provide for bi-ventricular pacing and/or cardiac resynchronization therapy (CRT), in addition to having LPs implanted in or on the right atrial (RA) chamber and the right ventricular (RV) chamber, a further LP can be implanted in or on the left ventricular (LV) chamber. It is also possible that a single LP be implanted within a patient, e.g., in or on the RV chamber, the RA chamber, or the LV chamber, but not limited thereto. It would also be possible for more than one LP to be implanted in or on a same cardiac chamber. The ICD 106 is shown as being connected to a lead 103, on which is located multiple electrodes. In FIG. 1, the electrically conductive canister (aka housing or case) of the ICD (or a portion thereof) provides a can or case electrode 125a. The lead 103 is shown as including a distal ring (or tip) electrode 125b, a proximal ring electrode 125c, a distal coil electrode 125d (also known as a parasternal coil electrode 125d) and a proximal coil electrode 125e (also known as a traverse coil electrode 125e). The lead 103 can include more or less electrodes than shown. It is also possible that the ICD 106 be connected to more than one lead and/or to a patch electrode.

In some embodiments, one or more of the LPs 102a, 102b can be co-implanted with the ICM 104 and/or the ICD 106. In such embodiments, the ICM 104 and/or the ICD 106 are examples of other types of IMDs that may need to communicate with the external device 109, such as an external programmer, from time to time. The ICM 104 and/or the ICD 106 may utilize conductive communication to communicate with the LPs 102, with one another, as well as to communicate with the external device 109. It may alternatively or additionally be possible for the ICM 104 and/or the ICD 106 to utilize radio frequency (RF) communication and/or inductive communication to communicate with an external device, depending upon the specific implementation, and depending upon the capabilities of the external device. In certain embodiments, the various devices of the system 100 are only able to communicate with one another using conductive communication.

Each LP 102a, 102b uses two or more electrodes located within, on, or within a few centimeters of the housing of the pacemaker, for pacing and sensing at the cardiac chamber, for bidirectional conductive communication with one another, with the external device 109, the ICD 106, and/or the ICM 104. Such an ICM 104 can be intended for subcutaneous implantation at a site near the heart 101. The ICM 104 can include, for example, a pair of spaced-apart sense electrodes positioned with respect to a housing, wherein the sense electrodes provide for detection of far-field EGM signals, and can also be used for conductive communication with one or more other implanted devices, such as the LP(s) 102a and/or 102b and/or the ICD 106. Such an ICM can also include an antenna that is configured to wirelessly communicate with the external device 109, such as an external programmer or a remote monitor, in accordance with one or more wireless communication protocols (e.g., Bluetooth, Bluetooth low energy, Wi-Fi, etc.). The housing of the ICM 104 can include various other components such as: sense electronics for receiving signals from the electrodes, a microprocessor for processing the signals in accordance with algorithms, a loop memory for temporary storage of cardiac activity (CA) data, a device memory for long-term storage of CA data upon certain triggering events, sensors for detecting patient activity and a battery for powering components.

Each LP 102a, 102b and/or other type of IMD can transmit an advertisement sequence of pulses using at least two electrodes of the IMD (e.g., LP) from time to time so that an external device (e.g., an external programmer, or a remote monitor) that has or is communicatively coupled to external electrodes that are in contact with the patient (within which the LP(s) and/or other IMD(s) is/are implanted) can detect the presence of the IMD(s) and optionally establish a communication session with one or more IMD(s). For a more specific example, an LP (or other type of IMD) can transmit an advertisement sequence of pulses every specified number of cardiac cycles (e.g., every eight cardiac cycles), or every specified period of time (e.g., every 5 seconds), but not limited thereto. In accordance with certain embodiments, the advertisement sequence of pulses is a predetermined sequence of pulses that indicates to an external device (e.g., an external programmer, or a remote monitor) that an LP (or other type of IMD) is implanted within a patient. The advertisement sequence of pulses can also be referred to as a sniff sequence of pulses, or more succinctly as a sniff. In accordance with certain, an external device (e.g., 109) can use the sniff pulses to identify which one of a plurality of communication vectors is a preferred conductive communication vector for communicating with the IMD that transmitted the sniff pulses. For example, where the external device has or is communicatively coupled to three external electrodes, i.e., first, second, and third external electrodes, the external device can test and select among first, second, and third subsets of the external electrodes, wherein the first subset includes the first and second external electrodes, the second subset includes the first and third external electrodes, and the third subset includes the second and third external electrodes.

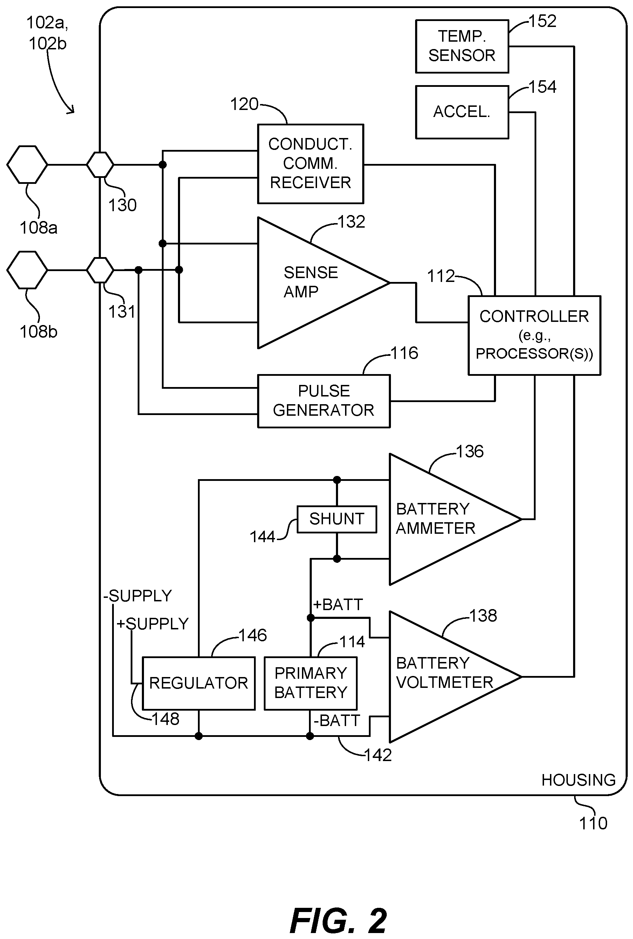

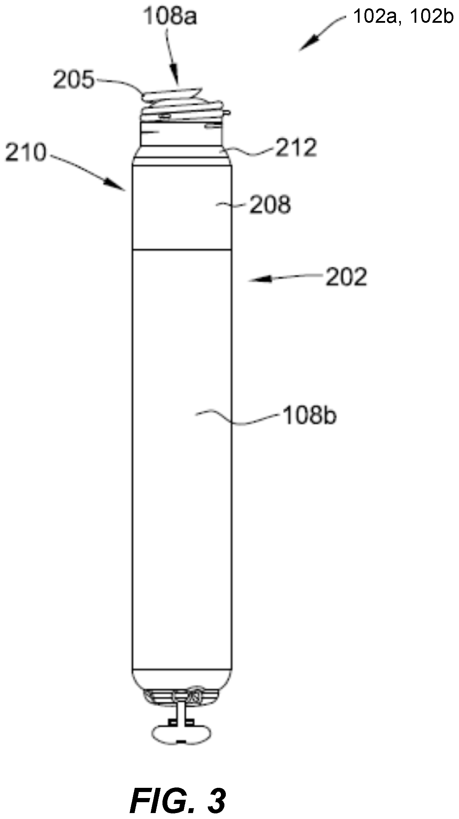

Referring to FIG. 2, a block diagram shows an example embodiment for portions of the electronics within the LPs 102a, 102b configured to provide conductive communication through the same electrodes that are used for cardiac pacing and/or sensing. Each of the LPs 102a, 102b includes two leadless electrodes configured for delivering cardiac pacing pulses, sensing evoked and/or natural cardiac electrical signals, and unidirectional and/or bidirectional conductive communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes, depending upon implementation. However, unless stated otherwise, for much of the following description it is assumed that each LP 102 includes only two electrodes, aka, a pair of electrodes.

In FIG. 2, each of the LPs 102a, 102b is shown as including a conductive communication receiver 120 that is coupled to the electrodes 108 and configured to receive conductive communication signals from the other LP 102, the ICM 104 and/or the ICD 106, but not limited thereto. The conductive communication receiver 120 and the electrodes 108 can also be used to receive conductive communication signals from the external device 109, and/or another type of external device. Although one receiver 120 is depicted in FIG. 2, in other embodiments, each LP 102a, 102b may also include one or more additional receivers. For example, each LP 102 can include a low frequency (LF) receiver and a high frequency (HF) receiver, as described, e.g., in U.S. Patent No. 11,090,497. As will be described in additional detail below, the pulse generator 116 can function as a transmitter that transmits conductive communication signals using the electrodes 108, under the control of the controller 112. The controller 112 can modulate and/or encode such pulses, or modulation and/or encoding of pulses can be performed external to the controller 112, yet at least in part under the control of the controller 112, depending on the specific implementation.

In certain embodiments, the LPs 102a, 102b may communicate over more than just first and second communication channels 105 and 107. In certain embodiments, the LPs 102a, 102b may communicate over one common communication channel 105. More specifically, the LPs 102a and 102b can communicate conductively over a common physical channel via the same electrodes 108 that are also used to deliver pacing pulses. Usage of the electrodes 108 for conductive communication enables the one or more LPs 102a, 102b to perform antenna-less and inductive coil-less communication. Where multiple implantable devices (such as the LPs 102a and 102b) communicate with one another using conductive communication, such conductive communication can be referred to as implant-to-implant (i2i) conductive communication, or more succinctly, as i2i conductive communication.

Optionally, an LP (or other IMD) that receives any conductive communication signal from another LP (or other IMD) or from a non-implanted device (e.g., a programmer or other external device) may transmit a receive acknowledgement indicating that the receiving LP (or other IMD, or external device) received the conductive communication signal. In certain embodiments, where an IMD expects to receive a conductive communication signal within a window, and fails to receive the conductive communication signal within the window, the IMD may transmit a failure-to-receive acknowledgement indicating that the receiving IMD failed to receive the conductive communication signal. Other variations are also possible and within the scope of the embodiments described herein. Each conductive communication signal can include one or more sequences of conductive communication pulses. In accordance with certain embodiments, conductive communication pulses are transmitted during cardiac refractory periods that are identified or detected by the LP(s) and/or other IMD(s). In accordance with certain embodiments, conductive communication pulses are sub-threshold, i.e., they are below the cardiac capture threshold for the patient.

The LPs 102a, 102b can exchange event messages within i2i conductive communication signals to enable synchronized therapy and additional supportive features (e.g., measurements, etc.). To maintain synchronous therapy, each of the LPs 102a, 102b is made aware (through the event messages) when an event occurs in the chamber containing the other LP 102a, 102b. Example additional details of i2i event messages that are sent between LPs 102 are provided in U.S. Patent No. 11,918,817.

For synchronous event signaling, LPs 102a and 102b may maintain synchronization and regularly communicate at a specific interval. Synchronous event signaling allows the transmitter and receiver in each LP 102a,102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20µs, where the Tx/Rx time slots occur periodically (e.g., every 10-20ms). Such time slots can also be referred to as windows.

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, a LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or an external device 109.

In accordance with certain embodiments herein, the external device 109 may communicate over a programmer-to-LP channel, with LPs 102a, 102b utilizing the same communication scheme. The external device 109 may listen to the event message transmitted between LPs 102a, 102b and synchronize programmer to implant communication such that the external device 109 does not transmit communication signals 113 until after an implant to implant messaging sequence is completed.

In some embodiments, an individual LP 102 can comprise a hermetic housing 110 configured for placement on or attachment to the inside or outside of a cardiac chamber and at least two leadless electrodes 108 proximal to the housing 110 and configured for conductive communication with at least one other device within or outside the body. Depending upon the specific implementation, and/or the other device with which an LP is communicating, the conductive communication may be unidirectional or bidirectional.

FIG. 2 depicts a single LP 102a (or 102b) and shows the LP's functional elements substantially enclosed in a hermetic housing 110. The LP 102a (or 102b) has at least two electrodes 108 located within, on, or near the housing 110, for delivering pacing pulses to and sensing electrical activity from the muscle of the cardiac chamber, and for conductive communication with at least one other device within or outside the body. Hermetic feedthroughs 130, 131 conduct electrode signals through the housing 110. The housing 110 contains a primary battery 114 to supply power for pacing, sensing, and communication. The housing 110 also contains a sense amplifier 132 for sensing cardiac activity from the electrodes 108, a receiver 120 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example a battery current monitor 136 and a battery voltage monitor 138, and can contain circuits for controlling operations in a predetermined manner.

The electrodes 108 can be configured to communicate bidirectionally among the multiple leadless cardiac pacemakers, the implanted ICD 106 and/or the implanted ICM 104 to coordinate pacing pulse delivery and optionally other therapeutic or diagnostic features using messages that identify an event at an individual pacemaker originating the message and a pacemaker receiving the message can react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more LPs, the ICD 106, and/or the ICM 104 and transmit data including designated codes for events detected or created by an individual pacemaker. Individual pacemakers can be configured to issue a unique code corresponding to an event type and a location of the sending pacemaker. The electrodes can also be used to transmit and/or receive conductive communication signals from an external device, e.g., 109.

As shown in FIG. 2, each LP 102a, 102b can comprise two (or more) leadless electrodes 108 configured for delivering cardiac pacing pulses, sensing evoked and/or natural cardiac electrical signals, and bidirectionally communicating with one another and/or the co-implanted ICD 106. As shown in FIG. 2, the primary battery 114 has positive terminal 140 and negative terminal 142. Current from the positive terminal 140 of primary battery 114 flows through a shunt 144 to a regulator circuit 146 to create a positive voltage supply 148 suitable for powering the remaining circuitry of the pacemaker 102. The shunt 144 enables the battery current monitor 136 to provide the controller 112 with an indication of battery current drain and indirectly of device health. The illustrative power supply can be a primary battery 114. The LP is also shown as including a temperature sensor 152 and an accelerometer 154.

In various embodiments, each LP 102a, 102b can manage power consumption to draw limited power from the battery, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 in one LP 102 can access signals on the electrodes 108 and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

FIG. 3 shows an example form factor of an LP 102a, 102b. The LP can include a hermetic housing 202 (110) with electrodes 108a and108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 2. The housing can also include an electronics compartment 210 within the housing that contains the electronic components necessary for operation of the pacemaker, including, e.g., a pulse generator, receiver, a battery, and a processor for operation. The hermetic housing 202 can be adapted to be implanted on or in a human heart, and can be cylindrically shaped, rectangular, spherical, or any other appropriate shapes, for example. The housing can comprise a conductive, biocompatible, inert, and anodically safe material such as titanium, 316L stainless steel, or other similar materials. The housing 202 can further comprise an insulator disposed on the conductive material to separate electrodes 108a and 108b. The insulator can be an insulative coating on a portion of the housing between the electrodes, and can comprise materials such as silicone, polyurethane, parylene, or another biocompatible electrical insulator commonly used for implantable medical devices. In the embodiment of FIG. 3, a single insulator 208 is disposed along the portion of the housing between electrodes 108a and 108b. In some embodiments, the housing itself can comprise an insulator instead of a conductor, such as an alumina ceramic or other similar materials, and the electrodes can be disposed upon the housing.

As shown in FIG. 3, the pacemaker can further include a header assembly 212 to isolate electrodes 108a and 108b. The header assembly 212 can be made from PEEK, tecothane or another biocompatible plastic, and can contain a ceramic to metal feedthrough, a glass to metal feedthrough, or other appropriate feedthrough insulator as known in the art. The term metal, as used herein, also encompasses alloys that are electrically conductive. The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, the electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 2) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

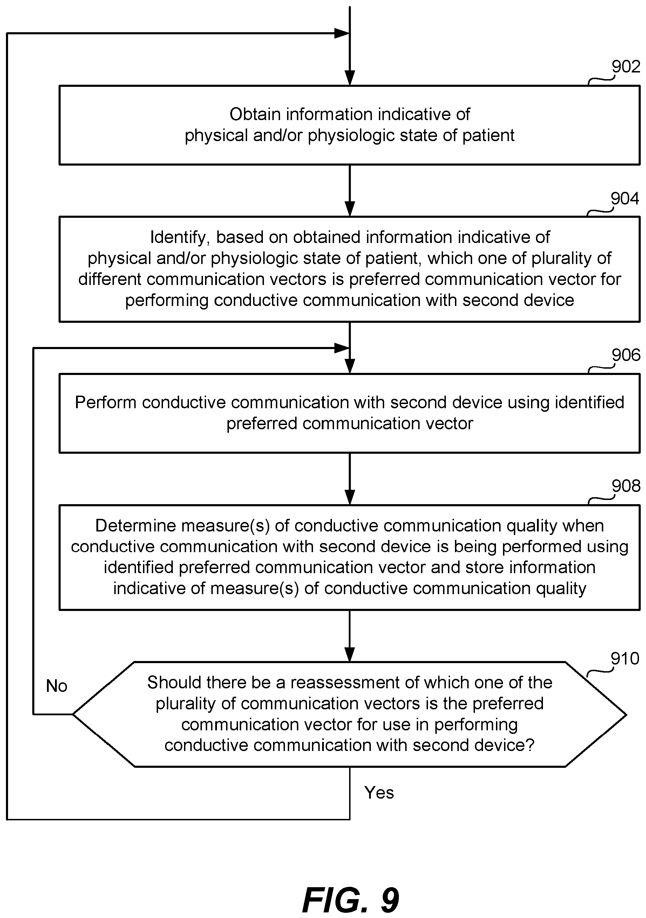

In FIGS. 1-3, each of the LPs 102 was shown and described as including two electrodes 108. In alternative embodiments, one or more of the LPs 102 can include more than two electrodes, e.g., three electrodes 108. Where an LP 102 includes at least three electrodes, the LP 102 can perform conductive communication using a plurality of different conductive communication vectors, wherein each of the plurality of different conductive communication vectors comprises a different combination of the at least three electrodes. Examples of an LP that includes at least three electrodes are described with reference to FIGS. 9A and 9B of commonly assigned U.S. Patent No. 10,642,705.

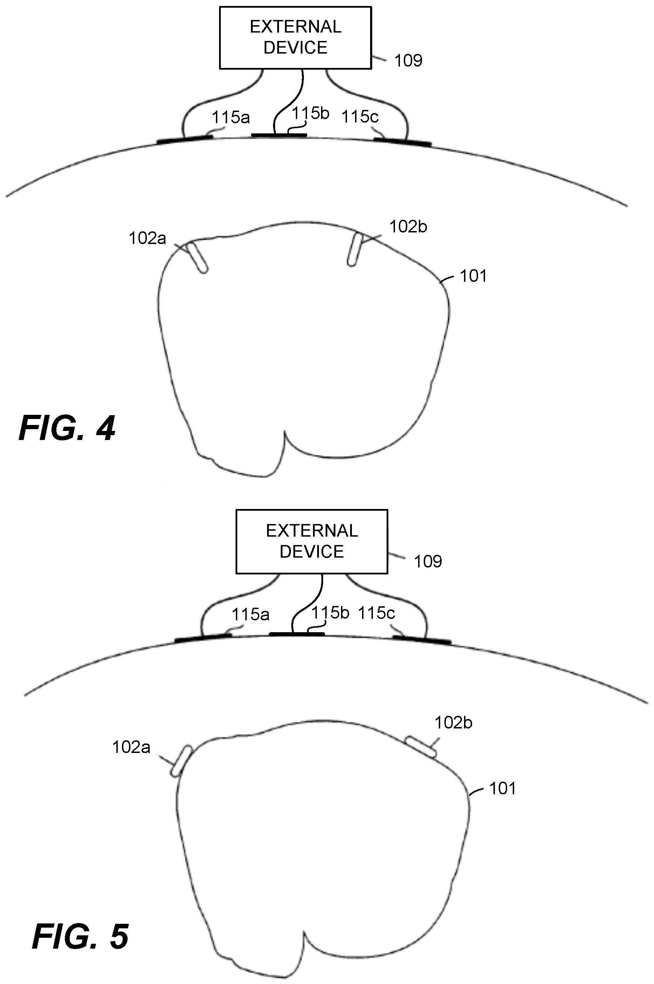

FIGS. 4 and 5 are schematic pictorial views depicting how an external device 109 coupled to three skin electrodes 115a, 115b, and 115c can communicate with the LP 102a, the LP 102b and/or one or more other IMD(s) via conductive communication, which is also referred to interchangeably herein as conducted communication. Such communication may take place via bidirectional communication pathways comprising a receiving pathway that decodes information encoded on pulses generated by one or more of the LPs 102a or 102b and conductive through body tissue to the external device 109. According to the illustrative arrangement, the bidirectional communication pathways can be configured for communication with multiple LPs 102a and 102b via two or more electrodes and conduction through body tissue.

The external device 109 is connected by a communication transmission channel and has transmitting and receiving functional elements for a bidirectional exchange of information with one or more IMDs, such as LP 102a and/or LP 102b. The communication channel includes three external electrodes 115a, 115b, and 115c which can be affixed or secured to the surface of the skin. From the point of the skin, the communication transmission channel is wireless, includes the ion medium of the intra- and extra-cellular body liquids, and enables electrolytic-galvanic coupling between the external electrodes, which can also be referred to as surface electrodes, and the LPs, or more generally, IMDs. The bidirectional communication pathways can further comprise a transmitting pathway that passes information from the external device 109 to one or more of the LPs 102a and/or 102b by direct conduction through the body tissue by modulation that avoids skeletal muscle stimulation using modulated signals at a frequency in a range from approximately 10 kHz to 100 kHz, or at higher frequencies. For example, p2i communication signals may be transmitted at a center frequency (fc) of 500 kHz.

Information transmitted from the external device 109 to the implanted LPs is conveyed by modulated signals at the approximate range of 10 kHz to 100 kHz which is a medium-high frequency, or at higher frequencies. The signals are passed through the communication transmission channel by direct conduction. A modulated signal in the frequency range has a sufficiently high frequency to avoid any depolarization within the living body which would lead to activation of the skeletal muscles and discomfort to the patient. The frequency is also low enough to avoid causing problems with radiation, crosstalk, and excessive attenuation by body tissue. Thus, information may be communicated at any time, without regard to the heart cycle or other bodily processes. The use of other frequency ranges is also possible and within the scope of the embodiments described herein.

FIG. 4 depicts a sample configuration involving the external device 109 and two endocardially implanted LPs 102a and 102b. The external device 109 is physically connected to the skin surface via three external electrodes 115a, 115b and 115c (also referred to as surface electrodes), which can serve three functions. The external electrodes 115a, 115b and 115c can be referred to individually as an external electrode 115 (or a surface electrode 115), or collectively as external electrodes 115 (or surface electrodes 115). First, the electrodes 115 can be used to transmit encoded information from the external device 109 to the LPs 102 or other type(s) of IMD(s) using a modulated signal, e.g., at a medium frequency 10 kHz to 100 kHz. Second, the external electrodes 115 can be used to receive information from individual LPs 102 and/or other IMD(s) by detecting encoded information in the communication pulses output by the LP(s) 102 and/or other IMD(s). Third, the external electrodes 115 can receive or sense a surface electrocardiogram for display and analysis by the external device 109.

In FIG. 4, the two LPs 102a and 102b are implanted within the heart 101 endocardially. Alternatively, as shown in FIG. 5, the two LPs 102a and 102b can be implanted by affixing to the exterior surface of the heart 101. The external electrodes 115 and the external device 109 function similarly in arrangements shown in FIGS. 4 and 5 whether the LPs 102a and 102b are implanted endocardially or epicardially (on the external heart surface). No restriction is imposed that the LPs are all implanted inside or all implanted outside the heart. One or more may be implanted endocardially along with others implanted on the outer surface of the heart. The functioning of the external device 109 is substantially the same.

As explained above in the Background, a potential problem with using conductive communication signals to provide for communication between two or more IMDs and/or between an external device and one or more IMDs, is that the orientation of the IMD(s) can cause fading that can adversely affect conductive communication. Additionally, the locations of the external electrodes, which define a communication vector for the external device, may not provide for good communication signal quality between the external device and an IMD. These problems may be exacerbated when there is a need or desire for more than two IMDs to communicate with one another, or when there is a need for an external device to communicate with multiple (i.e., two or more) IMDs.

### Use of Multiple Conductive Communication Vectors

As can be appreciated from the above discussion, including the discussion in the Background, an IMD, such as an LP, is susceptible to non-uniform periodic changes in orientation throughout a cardiac cycle, which can affect the ability of the IMD to successfully perform conductive communication with another IMD and/or an external device, such as a programmer or a remote monitor. Additionally, a patient's posture, breathing patterns and activity, can also affect one or more IMDs ability to successfully perform conductive communication with another IMD, and/or with an external device (e.g., an external programmer or a remote monitor).

Certain embodiments of the present technology described herein can be used improve (and preferably optimize) conductive communication signal quality between multiple IMDs, e.g., between a pair of LPs, between an LP and an NV-ICD, or between an LP and an ICM, but not limited thereto. Certain embodiments of the present technology described herein can alternatively or additionally be used improve (and preferably optimize) conductive communication signal quality between an external device (e.g., an external programmer or a remote monitor) and each of one or more IMDs.

Certain such embodiments relate to an optimization process for electrode pair selection, which may be performed in real-time through assessment of relative anatomical positions of multiple devices involved in i2i or p2i conductive communication and reconfiguration of communication vectors to those which previously resulted in the best i2i or p2i conductive communication performance for that positional arrangement. Furthermore, transmitter power consumption may be reduced (and preferably minimized) through receipt of a transmission via multiple vectors with disagreement in the decoded bitstream among the multiple vectors being resolved through identification of common bits in the majority of vectors. Furthermore, the data encoding protocol may be optimized to reduce (and preferably minimize) power consumption and/or bit-error-rate through assessment of the aforementioned parameters in various data encoding protocols.

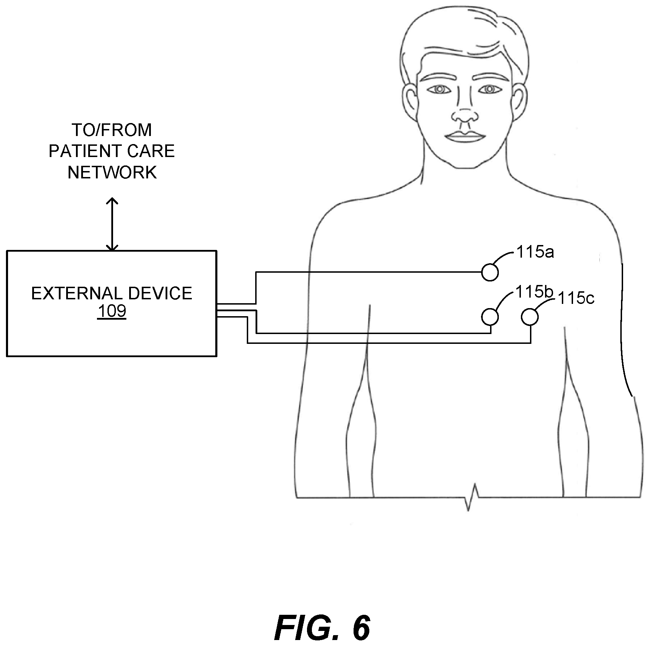

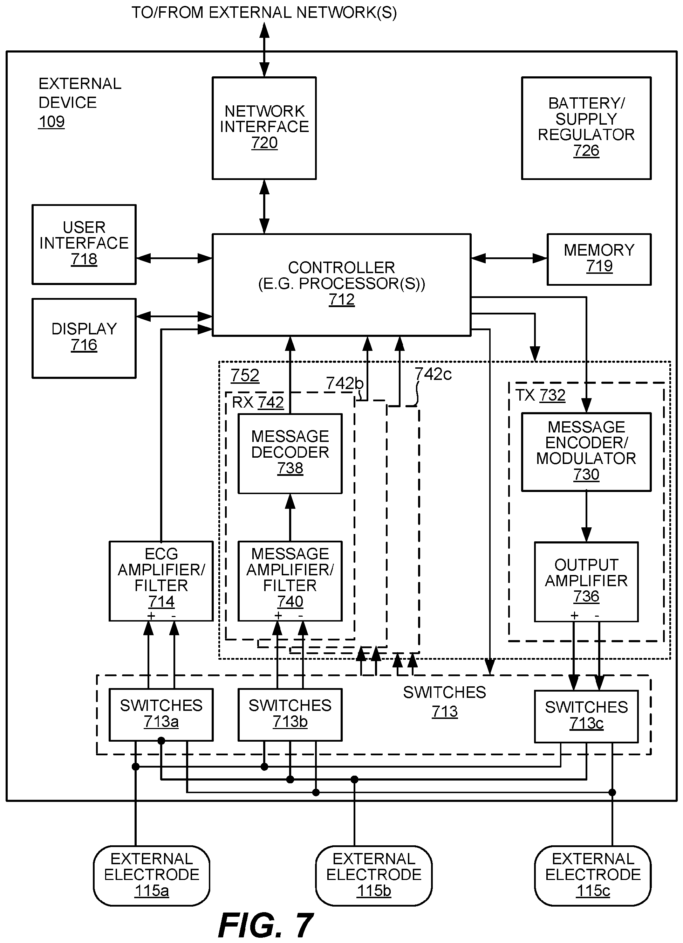

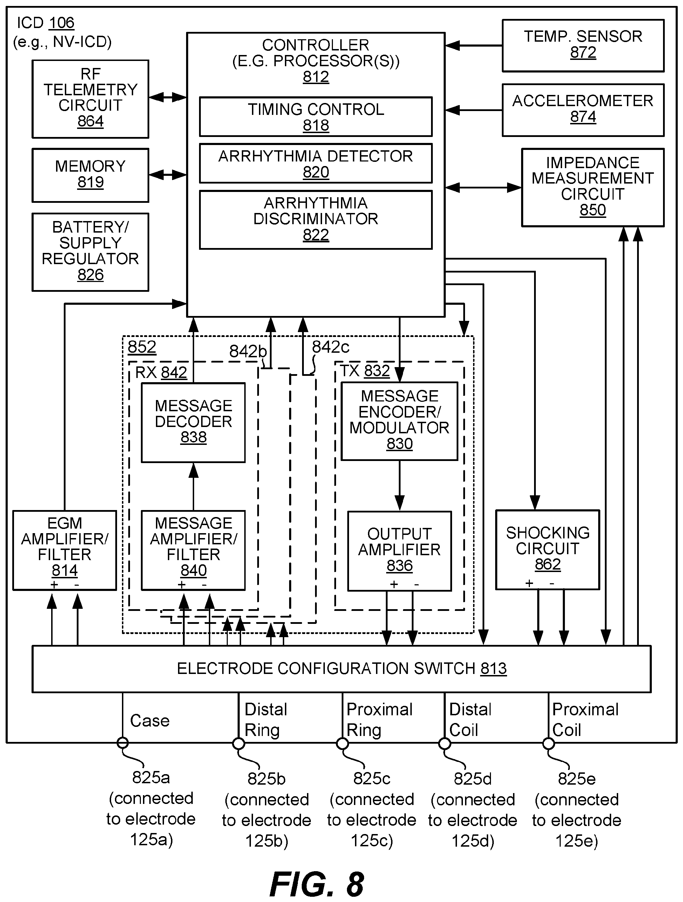

Certain embodiments described herein assess the conductive communication signal quality for a given conductive communication vector, and criteria are used to determine a preferred conductive communication vector for use in conductive communication between a pair of devices. In order for a device to be able to select among different conductive communication vectors for performing conductive communication with another device, the device should include or be communicatively coupled to at least three electrodes. An example of an external device including or communicatively coupled to at least three external electrodes is shown in and described with reference to FIGS. 6 and 7. An example of an IMD including or communicatively coupled to at least three implantable electrodes is shown in and described with reference to FIG. 8.

Referring to FIG. 6, the external device 109 is shown as being communicatively coupled to three external electrodes 115a, 115b, and 115c, which can be referred to collectively as the external electrodes 115, or individually as an external electrode 115. In FIG. 6 the external electrodes 115 are shown as being in contact with the chest of a patient, however, that need not be the case. Alternatively, one or more external electrodes can be in contact with the back of the patient, and/or with limbs or digits of the patient, but are not limited thereto. The external electrodes 115 can be physically separated from one another to thereby enable each of the electrodes to be independently placed in contact with the patient's skin at any desired location. Alternatively, the external electrodes 115, while electrically isolated from one another, can be physically attached to a same patch or substrate, e.g., a triangular or Y-shaped patch, or the like, that can be configured to be placed on a patient's chest or back, but not limited thereto. The external device 109 can optionally be communicatively coupled to a remote patient care network, e.g., via one or more wired and/or wireless communication network(s). Example details of the external device 109 are described below with reference to FIG. 7. In certain embodiments, the external device 109 can receive conductive communication signals from IMD(s) but cannot send conductive communication signals to IMD(s), i.e., is only capable of unidirectional communications.

Referring to FIG. 7, shown therein is an example block diagram of the external device 109, which is configured to communicate with one or more IMDs implanted within a patient using conductive communication, wherein the external device 109 includes and/or is communicatively coupled to at least three external electrodes 115 that are in contact with the patient.

Where the external device 109 is an external programmer, the external device is capable of programming one or more IMDs, such as one or more LPs, an ICM and/or an ICD. The external device 109 can also be used to obtain diagnostic information from one or more IMDs. Where the external device 109 is a remote monitor, it may not be capable of programming any IMDs. The external device 109 is shown as including a controller 712, a display 716, a user interface 718, a network interface 720, and a battery/supply regulator 726. The battery and/or supply regulator 726 provides one or more constant voltages to the various components of the external device 109 during normal operation. The external device 109 is also shown as including an ECG amplifier and/or filter 714, a conductive communication receiver (RX) 742, and a conductive communication transmitter (TX) 732. The receiver 742, in this example embodiment, is shown as including a message amplifier and/or filter 740, and a message decoder 738, and is configured to receive conductive communication signals from one or more LPs (e.g., 102a and/or 102b), and provide a bitstream to the controller 712.

The controller 712, which is used to control the operation of the external device 109, can include, e.g., one or more processors (or equivalent control circuitry), RAM and/or ROM memory, logic and timing circuitry, state machine circuitry, and/or I/O circuitry, but is not limited thereto. The controller 712 can also include a clock circuit, or a separate clock circuit (not shown) can provide a clock signal to the controller 712.

In the embodiment shown in FIG. 7, the external device 109 is shown as being connected to three external electrodes 115a, 115b, and 115c, which can be referred to collectively as the electrodes 115, or individually as an electrode 115. The electrodes 115 are shown as being connected to switches 713, which are shown as including first, second, and third sets of switches 713a, 713b, and 713c. The switches 713 can also be referred to herein as an electrode configuration switch 713. The external electrodes 115 can be located on a housing of the external device 109, or can be separate from such a housing. Where the electrodes 115 are separate from a housing of the external device 109, each of the electrodes 115 can be attached to a separate respective wire, or the electrodes 115 can be attached to a further housing that is communicatively coupled to the external device 109 via one or more wires, or via a wireless connection, e.g., using Bluetooth or WiFi, but not limited thereto. Other variations are also possible and within the scope of the embodiments described herein. The external electrodes 115, as will be described in more detail below, can be used to transmit and receive conductive communication signals to/from one or more LPs, and/or one or more other types of IMDs, and optionally can also be used to sense an electrocardiogram (ECG).

The external electrodes 115 are intended to come into contact with the skin of a patient. For example, the external electrodes can be skin electrodes that are configured to be attached to a patient's torso (e.g., chest and/or back) via an adhesive and/or gel. For another example, the external electrodes 115 can be configured to be touched by one or more digits on each hand of a patient, or to come into contact with a patient's wrist, a patient's limb, or a patient's chest, but are not limited thereto. A set of switches 713a is connected between the electrodes 115 and the ECG amplifier and/or filter 714, a set of switches 714b is connected between the electrodes 115 and the receiver 742, and a further set of switches 713c is connected between the electrodes 115 and the transmitter 732. The various sets of switches are controlled by the controller 712. In certain embodiments, the amplifiers and/or filters 714, 740, and 736 are each differential circuits that are intended to be connected to a pair of the electrodes 115 by the switches 713 under the control of the controller 712. For an example, the switches 713b can be controlled to connect any pair of the electrodes 115a, 115b, 115c to the message amplifier and/or filter 740. For an example, the switches 713b can connected the electrode 115a to a first input of the message amplifier/filter 740, and connect the electrode 115b to a second input of the message amplifier/filter 740, and not connect electrode 115c to any input of the message amplifier/filter 740. It is also possible that the switches can connect two electrodes 115 directly to one another. For an example, the switches 713b can connect the electrode 115a to a first input of the message amplifier/filter 740, and connect the electrodes 115b and 115c to one another and to a second input of the message amplifier/filter 740. Beneficially, connecting together two or more electrodes (e.g., 115b and 115c) to a same node (e.g., to the same input node of the message amplifier/filter 740) can effectively average or create a virtual vector which is between the two or more electrode locations, which enables sensing of a signal that is effectively an average of the signals detected at the two separate electrodes. This is an example of where a combination of the three electrodes 115a, 115b, and 115c includes all three of the electrodes, with the electrode 115a being separate from the other electrodes, and the electrodes 115b and 115c being electrically coupled to one another. The inclusion of three external electrodes 115 enables an ECG to be sensed at multiple vectors and/or enables selection from among the multiple vectors for conductive communication with one or more implanted IMDs so that conductive communication quality can be improved or maximized.

As noted above, the conductive communication receiver 742, which is shown as including the message amplifier and/or filter 740, and the message decoder 738, is configured to receive conductive communication signals from one or more IMDs. The message amplifier and/or filter 740 is configured to amplify and/or filter conductive communication signals received from an IMD. The amplifier portion can be used to increase the relatively small amplitudes of such conductive communication signals. The filter portion can be a high-pass filter or a bandpass filter adapted to separate an ECG signal from conductive communication signals. The message decoder 738 can be configured to decode conductive communication signals received from an IMD into a format that the controller 712 can understand. The specific type of decoding performed by the message decoder 738 can depend upon the specific coding of the conductive communication signals received from an IMD, e.g., on-off keying, frequency-shift keying, frequency modulation, or amplitude shift keying, but not limited thereto. It is also possible that the message decoder 738 be implemented as a comparator that compares the output of the message amplifier and/or filter 740 to a threshold, and outputs binary 1s and 0s based on results of the comparisons, and thereby provides a bitstream to the controller 712. The controller 712 can then perform the specific type of decoding that is dependent on how the conductive communication messages were encoded by the transmitting device.

In certain embodiments, the external device 109 includes one, two or more additional instances of the conductive communication receiver 742, with example additional instances of the conductive communication receiver labeled 742b and 742c. Each additional instance of the receiver 742 can include its own respective message amplifier and/or filter 740 and its own respective message decoder 738, and can provide its own bitstream to the controller 712. Using the switches 713, which are controlled by the controller 712, each of the different receivers (e.g., 742, 742b, and 742c) can be electrically coupled to a different pair or combination of the external electrodes 115a, 115b, 115c, and can thereby be electrically coupled to a different conductive communication vector.

The conductive communication transmitter 732 is configured to transmit (under the controller of the controller 712) conductive communication signals to one or more IMDs implanted within a patient. One example of a conductive communication signal that may be transmitted by the external device 109, such as an external programmer or a remote monitor, is an acknowledgement (ACK) sequence of conductive communication pulses, which informs one or more IMDs that the external device 109 is in proximity to the LP(s) and/or other types of IMD(s) and capable of receiving data (encoded into conductive communicate pulses) from the LP(s) and/or other types of IMD(s). The conductive communication signals can also be used to program, interrogate, and/or obtain notifications and/or other types of diagnostic information from one or more IMD(s).

The transmitter 732, in this example embodiment, is shown as including a message encoder and/or modulator 730 and an amplifier 736. The message encoder and/or modulator 730 can be configured to encode and/or modulate signals that are output from the controller 712 into a format that IMD(s) can understand. The specific type of encoding performed by the message encoder depends upon the specific type of encoding the IMD(s) can understand, e.g., on-off keying, frequency-shift keying, frequency modulation, or amplitude shift keying, but not limited thereto. The amplifier 736 is coupled to the encoder/modulator 730 to increase amplitudes of pulses included in a conductive communication signals to a level sufficient to enable one or more IMD(s) to receive conductive communication signals from the external device 109. The transmitter 732, because it outputs conductive communication pulses, can also be referred to as a pulse generator.

The conductive communication receiver(s) 742 and the conductive communication transmitter 732 are example components of conductive communication circuitry 752 of the external device 109. In FIG. 7, the switches 713 are located between the conductive communication circuitry 752 and the external electrodes 115a, 115b, and 115c that are part of or communicatively coupled to the external device 109. The switches 713, under the control of the controller 712, enable three or more different conductive communication vectors to be electrically coupled to the conductive communication circuitry 752, or portions thereof. While only three external electrodes 115 are shown in FIG. 7, it is possible that the external device 109 can include or be coupled to more than three external electrodes 115. For example, the external device 109 can include and/or be coupled to four, five, or six external electrodes 115, but not limited thereto, wherein the greater the number of external electrodes the greater the number of potential conductive communication vectors to test and select from.

The controller 712 may receive ECG data and optionally displays an ECG using the display 716 and can also display information included in other data acquired from the implanted IMD(s) acquired through the encoded pulses included in conductive communication signals, such as battery voltage, sensed cardiac signal amplitude, or other system status information. The controller 712 also can accept input from a user via a user interface 718, which can include, e.g., a keyboard and/or touch-screen, but is not limited thereto. The controller 712 can also communicate over a network interface 720 to other data entry or display units, such as a handheld computer or laptop/desktop unit. The network interface 720 can be cabled or wireless and can also enable communication to a local area network or the Internet for greater connectivity. More specifically, the network interface 720 can be used to send ECG data, diagnostic data, and other types of data collected from one or more IMD(s) to a patient care network associated with a medical group and/or facility. For more specific examples, the network interface can include a Bluetooth antenna, a WiFi antenna, and/or an Ethernet connection, but is not limited thereto.

The controller 712, which can include one or more processors, and/or the like, can execute operations based on firmware stored in non-volatile memory (Flash). The non-volatile memory can also be used to store parameters or values that are to be maintained when power is removed. The controller 712 can use volatile memory or random access memory (RAM) as general storage for information such as ECG data, status information, swap memory, and other data. Such memory, shown in FIG. 7 as memory 719, can be included within the controller 712 and/or can be communicatively coupled to the controller 712.

The external electrodes (e.g., 115) of an external device (e.g., 109) described herein can be used to sense ECG signals, as well as sense conductive communication signals output by one or more IMDs. It is also possible for the external electrodes of an external device to be used to receive electrogram (EGM) signal data included in conductive communication signals output by one or more IMDs, which EGM signal data can be received by the external device (using the external electrodes) and used to reproduce one or more electrogram signals that were sensed by one or more IMDs, wherein an EGM signal can also be referred to as an intracardiac electrogram (IEGM) signal. In addition to being able to communicate with one or more IMDs via conductive communication, the external device 109 can optionally have an antenna and RF communication capabilities that enable the external device 109 to wirelessly communicate with an implantable device, such as the ICM 104, via a wireless communication protocol, examples of which were discussed above. It would also be possible for the external device 109 to also include an inductive coil that enables the external device to perform inductive communication with an IMD that has such a capability.

The external device 109 can take many physical forms, but fundamentally it should be able to establish a conductive communication vector with the patient so that it can detect one or more IMDs' conductively communicated transmissions, decipher the communication protocol utilized by the IMD(s), and upload any acquired follow-up information to a patient care network, such as the Merlin.net^{™} patient care network operated by Abbott Laboratories (headquartered in the Abbott Park Business Center in Lake Bluff, Illinois).

For example, where the external device 109 has or is communicatively coupled to three external electrodes 115a, 115b and 115c, which can be referred to respectively as first, second, and third external electrodes, the external device can test and select among first, second, and third subsets of the external electrodes, wherein the first subset includes the first and second external electrodes (i.e., 115a and 115b), the second subset includes the first and third external electrodes (i.e., 115a and 115c), and the third subset includes the second and third external electrodes (i.e., 115b and 115c). In accordance with certain embodiments of the present technology, which are described below, the external device 109 can identify which one of a plurality of the subsets, or more generally, which one of the plurality of possible communication vectors, is a preferred conductive communication vector for communicating with an IMD. Further, as will be described in additional details below, where multiple IMDs are implanted within a patient, the external device can determine that different conductive communication vectors are preferred for different IMDs. However, it is also possible that the external device may determine that a same communication vector is preferred for communicating with two or more different IMDs.

Referring to FIG. 8, shown therein is an example block diagram of the ICD 106 that can communicate with one or more other IMDs within a patient using conductive communication, and/or to communicate with an external device (e.g., 109) using conductive communication and/or RF communication. For the purpose of this discussion, it is presumed that the ICD 106 is an NV-ICD, such as, but not limited to, a subcutaneous ICD (S-ICD). The NV-ICD 802 can communicate with one or more LPs 102 and/or an ICM 104. The ICD 106 is shown as including a controller 812, a battery/supply regulator 826, an ECG amplifier and/or filter 814, memory 819, a conductive communication receiver (RX) 842, a conductive communication transmitter (TX) 832, an RF telemetry circuit 864, a shocking circuit 862 and an electrode configuration switch 813. The battery and/or supply regulator 826 provides one or more constant voltages to the various components of the ICD 106. The receiver 842, in this example embodiment, is shown as including a message amplifier and/or filter 840, and a message decoder 838, and is configured to receive conductive communication signals from one or more LPs (e.g., 102a and/or 102b), an ICM 104 and/or an external device (e.g., 109). The controller 812, which is used to control the operation of the ICD 106, can include, e.g., one or more processors (or equivalent control circuitry), RAM and/or ROM memory, logic and timing circuitry, state machine circuitry, and/or I/O circuitry, but is not limited thereto. The controller 812 can also include a clock circuit, or a separate clock circuit (not shown) can provide a clock signal to the controller 812.

The controller 812 is shown as including a timing control module 818, an arrhythmia detector module 820, and an arrhythmia discriminator module 822, but can also include alternative and/or additional modules not specifically shown. The timing control module 818 can control the timing of the conductive communication windows, conductive communication pulses, shocking pulses, etc. The timing control module 818 may also be used for the timing of refractory periods, blanking intervals, noise detection windows, evoked response windows, alert intervals, marker channel timing, and so on. The arrhythmia detector 820 can be used to detect arrhythmias, and the arrhythmia discriminator 822 can be used to distinguish between different types of arrhythmias and/or to determine whether an arrhythmia detection should be classified as a false positive detection. For example, the arrhythmia detector 820 and/or the arrhythmia discriminator 822 can analyze a patient's heart rates and rhythms and determine, based on this analysis, whether the patient is experiencing fibrillation or impending fibrillation. The arrhythmia detector 820 and/or the arrhythmia discriminator 822 may also diagnose ventricular tachycardias and atrial tachycardias, but are not limited thereto, based on an EGM sensed by the ICD 106 and/or based on messages provided to the ICD 106 by one or more LPs 102 and/or an ICM 104.

In the embodiment shown in FIG. 8, the ICD 106 is shown as being electrically connected to five electrodes terminals 825a, 825b, 825c, 825d, and 825e, each of which is electrically connected to a respective electrode. For example, the electrode terminal 825a can be electrically connected to a case electrode (e.g., 125a), which is also known as a cannister or can electrode. The electrode terminals 825b, 825c, 825d, and 825e are electrically connected to electrodes on one or more leads (e.g., 103) that are coupled to the ICD 106. In an example embodiment, the electrode terminal 825b is electrically connected to a distal ring or tip electrode (e.g., 125b), the electrode terminal 825c is electrically connected to a proximal ring electrode (e.g., 125c), the electrode terminal 825d is electrically connected to a distal coil electrode (e.g., 125d) (also known as a parasternal coil electrode), and the electrode terminal 825e is electrically connected to a proximal coil electrode (e.g., 125e) (also known as a traverse coil electrode). The electrodes terminals 825a, 825b, 825c, 825d, and 825e, can be collectively referred to as electrode terminals 825, or individually as an electrode terminal 825. The electrodes 125a, 125b, 125c, 125d, and 125e can be collectively referred to as electrodes 125, or individually as an electrode 125. The electrodes 125 (via the electrode terminals 825) are connected to an electrode configuration switch 813, which can also be referred to herein as switches 813. The electrode configuration switch 813, under the control of the controller 812, can connect any two or more of the electrode 125 to the ECG amplifier and/or filter 814, the conductive communication RX 842, the conductive communication TX 832, or the shocking circuit 862. The electrodes 125, as will be described in more detail below, can be used to transmit and receive conductive communication signals to/from one or more LPs, and/or one or more other types of IMDs, and can also be used to sense an electrocardiogram (ECG).

In certain embodiments, the amplifiers and/or filters 814, 840, and 836 are each differential circuits that are intended to be connected to a pair of the electrodes 125 by the switches 813 under the control of the controller 812. For an example, the switches 813 can be controlled to connect any pair of the electrodes 125a, 125b, 125c, 118d, 118e to the message amplifier and/or filter 840. It is also possible that the switches 813 can connect two electrodes 125 directly to one another. For an example, the switches 813 can connect the electrode 125a to a first input of the message amplifier/filter 840, and connect the electrodes 125b and 125c to one another and to a second input of the message amplifier/filter 840. Beneficially, connecting together two or more electrodes (e.g., 125b and 125c) to a same node (e.g., to the same input node of the message amplifier/filter 840) can effectively average or create a virtual vector which is between the two or more electrode locations, which enables sensing of a signal that is effectively an average of the signals detected at the two separate electrodes.

The conductive communication receiver 842, which is shown as including the message amplifier and/or filter 840, and the message decoder 838, is configured to receive conductive communication signals from one or more LPs (e.g., 102a and/or 102b), an ICM 104 and/or an external device (e.g., 109). The conductive communication receiver 842 and the components thereof operates similar to the conductive communication receiver 742 and the components thereof, discussed above with reference to FIG. 7.

The message amplifier and/or filter 840 is configured to amplify and/or filter conductive communication signals received from another IMD or an external device (e.g., 109). The amplifier portion can be used to increase the relatively small amplitudes of such conductive communication signals. The filter portion can be a high-pass filter or a bandpass filter adapted to separate an ECG signal from conductive communication signals. The message decoder 838 can be configured to decode conductive communication signals received from another IMD or an external device into a format that the controller 812 can understand. The specific type of decoding performed by the message decoder 838 can depend upon the specific coding of the conductive communication signals received from another IMD or an external device, e.g., on-off keying, frequency-shift keying, frequency modulation, or amplitude shift keying, but not limited thereto. It is also possible that the message decoder 838 be implemented as a comparator that compares the output of the message amplifier and/or filter 840 to a threshold, and outputs binary 1s and 0s based on results of the comparisons, and thereby provides a bitstream to the controller 812. The controller 812 can then perform the specific type of decoding that is dependent on how the conductive communication messages were encoded by the transmitting device.

In certain embodiments, the ICD 106 includes one, two or more additional instances of the conductive communication receiver 842, with example additional instances of the conductive communication receiver labeled 842b and 842c. Each additional instance of the receiver 842 can include its own respective message amplifier and/or filter 840 and its own respective message decoder 838, and can provide its own bitstream to the controller 812. Using the switches 813, which are controlled by the controller 812, each of the different receivers (e.g., 842, 842b, and 842c) can be electrically coupled to a different pair or combination of the external electrodes 125a, 125b, 125c, 125d, and 125e, and can thereby be electrically coupled to a different conductive communication vector.

The conductive communication transmitter 832 is configured to transmit (under the controller of the controller 812) conductive communication signals to one or more IMDs implanted within a patient and/or an external device. The conductive communication signals can also be used to program, interrogate, and/or obtain notifications and/or other types of diagnostic information from one or more IMD(s). The transmitter 832, in this example embodiment, is shown as including a message encoder and/or modulator 830 and an amplifier 836. The conductive communication transmitter 832 and the components thereof operates similar to the conductive communication transmitter 842 and the components thereof, discussed above with reference to FIG. 7, and thus, need not be described in additional detail. The transmitter 832, because it outputs conductive communication pulses, can also be referred to as a pulse generator.

The conductive communication receiver(s) 842 and the conductive communication transmitter 832 are example components of conductive communication circuitry 852 of the ICD 106. In FIG. 8, the switches 813 are located between the conductive communication circuitry 852 and the electrodes 125a, 125b, 125c, 125d, and 125e that are part of or communicatively coupled to the ICD 106. The switches 813, under the control of the controller 812, enable three or more different conductive communication vectors to be electrically coupled to the conductive communication circuitry 852, or portions thereof. While five electrodes 125 are shown in FIG. 8, it is possible that more or less than five electrodes 125 are part of, or communicatively coupled to, the ICD 106.

The controller 812, which can include one or more processors, and/or the like, can execute operations based on firmware stored in non-volatile memory (Flash). The non-volatile memory can also be used to store parameters or values that are to be maintained when power is removed. The controller 812 can use volatile memory or RAM as general storage for information such as ECG data, status information, swap memory, and other data. Such memory, shown in FIG. 8 as memory 819, can be included within the controller 812 and/or can be communicatively coupled to the controller 812.

The switches 813 can also connect various combinations of the electrodes to an impedance measurement circuit 850. The impedance measurement circuit 850 includes inputs to collect multiple measured impedances between corresponding multiple combinations of electrodes. For example, the impedance measurement circuit 850 may collect a measured impedance for each or a subset of the active sensing vectors. Optionally, the impedance measurement circuit 850 may measure respiration or minute ventilation; measure thoracic impedance for determining shock thresholds: detect when the device has been implanted; measure stroke volume; and/or detect the opening of heart valves, etc.

The shocking circuit 862 generates shocking pulses of low (e.g., up to 0.5 joules), moderate (e.g., 0.5-10 joules), or high energy (e.g., 11 to 80 joules), as selected by the controller 812. Such shocking pulses are applied to a patient's heart through at least two shocking electrodes selected, for example, from the various electrodes 125. The electrodes selected to deliver a shock can be referred to herein as a shocking vector. Example shocking vectors include, but are not limited to, the following: the distal (also referred to as parasternal) coil electrode 125d to the proximal (also referred to as transverse) coil electrode 125e; the distal (also referred to as parasternal) coil electrode 125d and the proximal (also referred to as transverse) coil electrode 125e electrically coupled to one another (to form an anode of the vector) to the case (also known as canister, or can) electrode 125a (that provides the cathode of the vector); the distal ring (or tip) electrode 125b electrically coupled to the distal (also referred to as parasternal) coil electrode 125d (to form an anode of the vector) to the case (also known as canister, or can) electrode 125a (that provides the cathode of the vector); and the distal (also referred to as parasternal) coil electrode 125d to the case (also known as canister, or can) electrode 125a. Other variations are also possible and within the scope of the embodiments described herein.

The ICD 106 is also shown as including a temperature sensor 872 and an accelerometer 874, each of which is communicatively coupled to the controller 812. The temperature sensor 872 can be used by the controller 812 to determine an activity level of the patient within which the ICD 106 is implanted. The activity level of the patient can alternatively or additionally be determined based on one or more outputs of the accelerometer 874. The accelerometer 874 can alternatively, or additionally, be used by the controller 812 to determine the posture of the patient within which the ICD 106 is implanted.

The high level flow diagram of FIG. 9 will now be used to summarize certain methods for use by a first device that is configured to communicate with a second device using conductive communication, wherein the first device includes and/or is communicatively coupled to at least three electrodes (e.g., 115 or 125) and can perform the conductive communication using a plurality of different conductive communication vectors. In such embodiments, each of the plurality of different conductive communication vectors comprises a different combination of the at least three electrodes. In such embodiments, the second device includes and/or is communicatively coupled to at least two additional electrodes (e.g., 108) that can be used to perform the conductive communication. Additionally, in such embodiments, at least one of the first or second devices comprises an IMD implanted in a patient. For example, the first and second devices can be first and second IMDs. For a more specifical example, the first device can be an ICD (e.g., 106) including or communicatively coupled to electrodes 125a, 125b, 125c, 125d and 125e, and the second device can be an LP (e.g., 102) including electrodes 108a and 108b. For another example, the first device can be an external device (e.g., an external programmer or a remote monitor) communicative coupled to at least three electrodes (e.g., 115a, 115b and 115c), and the second device can be an IMD. Example types of the IMDs with which such methods can be used include, but are not limited to, LPs, NV-ICDs (e.g., S-ICDs), and ICMs. The steps described with reference to the flow diagram of FIG. 9 are presumed to be performed by the first device, which as noted above, can be an external device or an IMD. More specifically, such steps can be performed under the control of a controller (e.g., 712 or 812) of an external device or an IMD.

Referring to FIG. 9, step 902 involves obtaining information indicative of at least one of a physical or physiologic state of the patient. The information obtained at step 902 can be indicative of a posture of a patient, in which case, the information can be obtained from an accelerometer. Alternatively, or additionally, the information obtained at step 902 can be indicative of an activity level of the patient, in which case, the information can be obtained from an accelerometer and/or a temperature sensor of an IMD. In other words, the information indicative of the posture and/or activity level of the patient can be, or be based on, one or more signals output from an accelerometer and/or one or more signals output from a temperature sensor. Alternatively, or additionally, the information obtained at step 902 can be, or can be based on, an impedance measurement and/or an electrogram (EGM) or electrocardiogram (ECG) measurement. This is because impedance measurements are likely to change depending upon the specific posture of the patient, such that different impedance measurement signatures can be indicative of different posture. Similarly EGM or ECG measurements, such as EGM or ECG morphology signatures, typically change based on the posture of the patient. Additionally, a patient's heart rate (HR), which is detectable from an EGM or ECG, should change based on the activity level of the patient, and is an example of an EGM or ECG measurement that typically changes based on the activity level of the patient. These are just a few examples of the information indicative of at least one of a physical and/or physiologic state of the patient that can be obtained at step 902, which example are not intended to be all encompassing. The physical and/or physiologic state of a patient can alternatively or additionally be a state of at least one cyclical physiologic cycle, such as a cardiac cycle, a respiratory cycle, or another type of cyclical physiologic cycle. Other variations are also within the scope of the embodiments described herein.

Step 904 involves identifying which one of the plurality of different conductive communication vectors is a preferred conductive communication vector for communicating with the second device, based on the information indicative of the at least one of the physical or physiologic state of the patient. Step 904 can also be performed based on further information that was stored (e.g., in memory of the first device), which further information can be a table or other type of data store that associates different postures and/or activity levels with respective preferred conductive communication vectors. Such further information can specify, for example, that: when a patient is standing upright a first conductive communication vector provides for the best conductive communication quality with the second device; when the patient is supine a second conductive communication vector provides for the best conductive communication quality with the second device; when the patient is prone a third conductive communication vector provides for the best conductive communication quality with the second device; when the patient is in a right lateral recumbent (RLR) posture a fourth conductive communication vector provides for the best conductive communication quality with the second device; when the patient is in a left lateral recumbent (LLR) posture a fifth conductive communication vector provides for the best conductive communication quality with the second device; etc. The preferred conductive communication vector can alternatively, or additionally, be based on the activity level of the patient.

Step 906 involves performing the conductive communication with the second device using the preferred conductive communication vector. Referring briefly back to FIG. 7, where the first device is an external device, step 906 can include the controller 712 controlling the switches 713 to connect a subset of the electrodes 115 corresponding to the preferred conductive communication vector to the transmitter 732 and/or the receiver 742. Referring briefly back to FIG. 8, where the first device is an IMD, step 906 can include the controller 812 controlling the switches 813 to connect a subset of the electrodes 125 corresponding to the preferred conductive communication vector to the transmitter 832 and/or the receiver 842. Step 906 can also include outputting conductive communication pulses using the subset of electrodes corresponding to the preferred conductive communication vector and/or receiving conductive communication pulses using the subset of electrodes corresponding to the preferred conductive communication vector.

Still referring to FIG. 9, step 908 involves determining and storing one or more measures of conductive communication quality with the second device associated with performing the conductive communication using the preferred conductive communication vector identified at step 904. Example measures of conductive communication quality that can be determined for a communication vector include, but are not limited to: a noise floor associated with the conductive communication vector, a measure of amplitude of at least a portion of a conductive communication signal received by the first device from the second device using the conductive communication vector, a measure of amplitude of at least a portion of a conductive communication signal received by the first device from the second device, a magnitude of at least a portion of a conductive communication signal received by the first device from the second device after rectification and integration thereof, a magnitude of at least a portion of a conductive communication signal received by the first device from the second device after rectification and integration thereof, a signal-to-noise ratio (SNR) of at least a portion of a conductive communication signal received by the first device from the second device, a total energy of at least a portion of a conductive communication signal received by the first device from the second after rectification and integration thereof, a bit-error-rate (BER) associated with at least a portion of a conductive communication signal received by the first device from the second device. Other variations are also possible and within the scope of the embodiments described herein.

Step 910 involves determining whether there should be a reassessment of which one of the plurality of communication vectors is the preferred conductive communication vector for the first device to use when communicating with the second device. Step 910 can be performed by the first device, or more specifically a controller (e.g., 712 or 812) thereof, while or after communicating with the second device using the preferred conductive communication vector for communicating with the second device. If the answer to the determination at step 910 is No, then flow returns to step 906. If the answer to the determination at step 906 is Yes, then flow returns to step 902, and steps 902 and 904 are performed again.

In accordance with certain embodiments, step 910 can involve determining whether an indicator of conductive communication quality associated with the preferred conductive communication vector for communicating with the second device has fallen below a corresponding threshold. If the answer is No then flow returns to step 906, and if the answer is Yes then flow returns to step 902. Alternatively, or additionally, step 910 can involve determining whether the first device has lost conductive communication with the second device. Alternatively, or additionally, step 910 can involve determining whether a specified period of time (e.g., 1 minute, 3 minutes, 10 minutes, 1 hour, or the like) has elapsed since the preferred conductive communication vector for communicating with the second device was most recently identified. If the answer is No then flow returns to step 906, and if the answer is Yes then flow returns to step 902. Alternatively, or additionally, step 910 can involve determining whether a posture and/or activity level of the patient (or more generally, a physical and/or physiologic state of the patient) has changed since the preferred conductive communication vector for communicating with the second device was most recently identified. Other variations are also possible and within the scope of the embodiments described herein.

As one or more IMDs may undergo transient changes in orientation during cyclic/periodic physical/physiologic phenomena that generally result in force acting on the IMD (e.g. cardiac cycle, respiratory cycle, exercise, eddy currents generated by turbulent blood flow, vibration due to use of motorized equipment, etc.), the direction of a voltage potential gradient resulting from conductive communication may be of great importance. As such, conductive communication may be suboptimal when the voltage potential gradient is applied across only one pair of electrodes through all phases of various cyclic/periodic physical/physiologic phenomena. Certain embodiments of the present technology try to resolve that issue by utilizing a method for automated conductive communication optimization which relies on multi-vector communication within a single cycle of a cyclical physical/physiologic phenomena.

As noted above, the physical and/or physiologic state of a patient can be a state of a cardiac cycle, a respiratory cycle, or another type of cyclical physiologic cycle. Where that is the case, the obtaining at step 902, the identifying at step 904, and the performing at step 906 can be performed two or more times (i.e., at least twice) during a same cyclical physiologic cycle (e.g., the same cardiac or respiratory cycle) such that the preferred conductive communication vector changes within the same cyclical physiologic cycle. This can result in at least two of the plurality of different conductive communication vectors being used to perform the conductive communication with the second device during the same cyclical physiologic cycle. For example, there can be a first preferred conductive communication vector that is identified and used during the systole phase of a cardiac cycle, and second preferred conductive communication vector that is identified and used during the diastole phase of the cardiac cycle. For another example, there can be a first preferred conductive communication vector that is identified and used during the inspiratory phase of a respiratory cycle, and second preferred conductive communication vector that is identified and used during the expiratory phase of the respiratory cycle. Other variations are also within the scope of the embodiments described herein. In such example embodiments, a device can periodically change between different preferred conductive communication vectors during a periodic cyclical physical and/or physiologic state of the patient in order to mitigate the probability of conductive communication being unsuccessful.

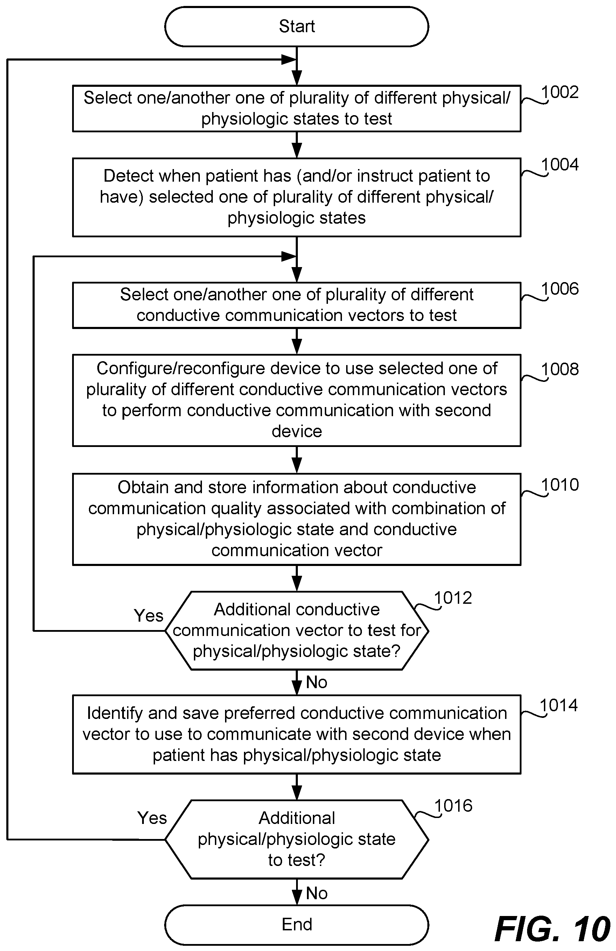

The high level flow diagram of FIG. 10 will now be used to describe a method for testing various different combinations of conductive communication vectors and physical and/or physiologic states of a patient, so that a respective preferred conductive communication vectors can be identified for each of a plurality of different physical and/or physiologic states of the patient. Such a method can be performed, for example, by an external programmer that includes and/or is communicatively coupled to at least three skin electrodes when a patient visits a clinic for the purpose of uploading data that has been stored within the IMD(s) and/or reprogramming the IMD(s), but not limited thereto. It would also be possible that the method is performed by a remote monitor that includes and/or is communicatively coupled to at least three skin electrodes. It is also possible that the method is performed by an IMD, e.g., an ICD (e.g., 106) that includes and/or is communicatively coupled to at least three implanted electrodes. Other variations are also possible and within the scope of the embodiments described herein.

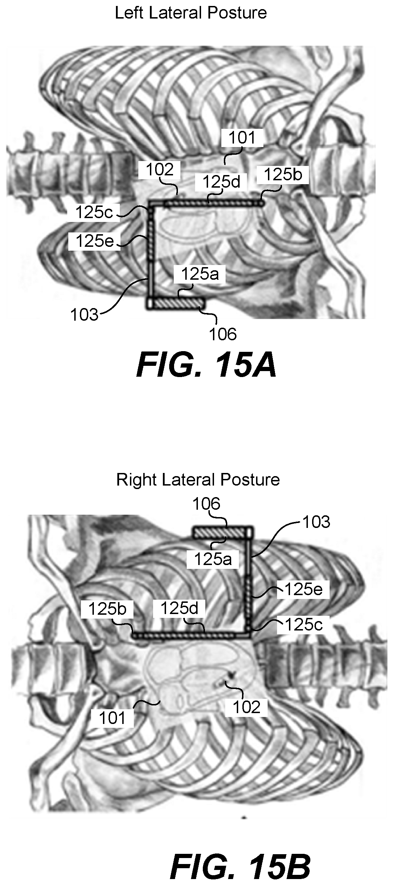

Referring to FIG. 10, step 1002 involves selecting one of a plurality of different physical and/or physiologic states of a patient, wherein a physical and/or physiologic state can also be referred to more succinctly as a physical/physiologic state. Step 1004 involves detecting when a patient has the selected physical/physiologic state and/or instructing the patient to have the selected physical/physiologic state. The selected physical/physiologic state can be or include a specific posture and/or activity level of the patient, but is not limited thereto. Example details about how a patient's posture and activity level can be determined are discussed above with reference to step 902. The posture of the patient can be detected, e.g., using an accelerometer and/or the patient can be instructed to assume a certain posture. The activity level of the patient can be detected using an accelerometer and/or a temperature sensor, but is not limited thereto. Alternatively, or additionally, the posture and/or activity level, and/or other type of physical/physiologic state can be determined based on an impedance measurement and/or an EGM measurement. The physical and/or physiologic state of a patient can alternatively or additionally be a state of at least one cyclical physiologic cycle that includes at least one of a cardiac cycle, a respiratory cycle, or another type of cyclical physiologic cycle. Other variations are also possible and within the scope of the embodiments described herein. FIGS. 15A and 15B, described later below, show an example of how the position of an LP 102, relative to electrodes of and/or coupled to an NV-ICD, can change depending upon the posture of the patient in which the LP and NV-ICD are implanted.

Still referring to FIG. 10, step 1006 involves selecting one of a plurality of different conductive communication vectors to use for performing conductive communication with a second device. Step 1008 involves configuring the device (e.g., controlling switches 713 or 813) to use the selected conductive communication vector to perform conductive communication with the second device. Step 1010 involves obtaining and storing information (e.g., in memory 719 or 819) about the conductive communication quality associated with the combination of the physical/physiologic state and the conductive communication vector. Example measures of conductive communication quality that can be determined and stored were discussed above, and thus, need not be repeated.

Step 1012 involves determining whether there is at least one additional conductive communication vector to test for the physical/physiologic state. If the answer to the determination at step 1012 is Yes, then flow returns to step 1006 and the device is reconfigured to test another conductive communication vector at a further instance of step 1008, and store information for the combination of the physical/physiologic state and the other conductive communication vector at another instance of step 1010.

If the answer to the determination at step 1012 is No, then flow goes to step 1014. Step 1014 involves determining (and storing information specifying) a preferred conductive communication vector for the physical/physiologic state of the patient. This can involve identifying which vector provided the best conductive communication quality when the patient has the physical/physiologic state. This way, at a later point in time when the patient has that same physical/physiologic state, the preferred conductive communication vector can be selected and used for performing conductive communication quality with the second device.

At step 1016 there is a determination of whether there is at least one additional physical/physiologic state to test. If the answer to the determination at step 1016 is Yes, then flow returns to step 1002 and another physical/physiologic state is tested, so that a respective preferred conductive communication vector can be determined and stored for each of a plurality of different physical/physiologic states of the patient. Thereafter, the preferred conductive communication vectors identified at instances of step 1014, for which information is saved, can be used, e.g., whenever step 904 in FIG. 9 is performed, but not limited thereto.

The method described with reference to FIG. 10 can be performed under the control of a controller (e.g., 712 or 812) of a device, where such a controller can include one or more processors. If the device that performs the method described with reference to FIG. 10 may perform conductive communication with more than one other device, e.g., with a second device and a third device, then the method described with reference to FIG. 10 can be performed by the device for the second device, and then separately by the device for the third device. This may result in the device, for a certain physical/physiologic state of the patient, identifying one conductive communication vector that is preferred to use when performing conductive communication with the second device, and identifying a second (different) conductive communication vector that is preferred to use when performing conductive communication with the third device, etc.

Unless stated otherwise, it is presumed that a same conductive communication vector is used by a device for both transmitting conductive communication signals to the second device and receiving conductive communication signals from the second device. Accordingly, where a preferred conductive communication is identified and used for performing conductive communication with a second device, it is presumed that the preferred conductive communication is used for both transmitting conductive communication signals to the second device and receiving conductive communication signals from the second device. However, in certain embodiments, one vector may be identified and used as the preferred conductive communication vector for transmitting conductive communication signals to the second device, while a different vector may be identified and used as the preferred conductive communication vector for receiving conductive communication signals from the second device. Indeed, it may be that for a certain physical/physiologic state of the patient a common vector is the preferred vector for both transmitting and receiving conductive communication signals to/from the second device, while for another physical/physiologic state of the patient the preferred vector for transmitting conductive communication signals to the second device differs from the preferred vector for receiving conductive communication signals from the second device.

Certain embodiments of the present technology can be used to avoid any uncomfortable muscle stimulation that could potentially result for transmission of conductive communication signals by an NV-ICD, or potentially another type of IMD. More specifically, testing can be performed during an implant procedure and/or thereafter to determine a conductive communication threshold voltage that results in patient discomfort due to muscle stimulation, and information indicative thereof can be stored in a memory of an IMD. The IMD can then purposely avoid transmitting conductive communication signals above or near (e.g., within a specified tolerance of) the conductive communication threshold voltage that resulted in patient discomfort due to muscle stimulation. In certain such embodiments, an IMD can switch to using another vector for transmitting conductive communication signals when conductive communication is unsuccessful or has an unacceptable quality at or near the conductive communication threshold voltage, in lieu of continuing to increase an output voltage using that vector. Alternatively, or additionally, the IMD can switch to using another vector for transmitting conductive communication signals if an evoked response of skeletal muscle is detected by a device, e.g., from a sensed electromyography (EMG).

Certain embodiments of the present technology further augment the conductive communication process by modulating the transmission timing (aka timing scheme) used with one or more conductive communication vectors as part of the conductive communication process in order to mitigate the probability that conductive communication may be performed via a suboptimal vector for a given intracardiac IMD in one phase of a cyclic physical/physiological phenomena, and, then performed again via a different suboptimal vector during another phase of the same cycle. Modulation of time between conductive communication message transmissions for each electrode pair may resolve this issue since the practice will change the phase of the physical/physiologic event in which the transmission from each electrode vector occurs. Furthermore, such embodiments may be enhanced by ceasing timing modulation for a conductive communication vector when conductive communication quality is acceptable (e.g., above a corresponding threshold) without timing modulation. In certain such embodiments, timing modulation may cease for one vector but may persist for one or more other vectors until conductive communication quality as assessed by one or more IMDs receiving the transmission is determined to be acceptable under the present timing configuration, the transmission is complete, or a number of transmissions have been attempted which meet a timeout threshold. In certain embodiments the practice of modulating time between conductive communication message transmissions for each vector as part of a multi-vector communication protocol may be utilized only when conductive communication quality has declined below a corresponding threshold, or when an arrythmia has been detected, or when noise is detected using one or more vectors for receiving a transmission.

The transmission timing that is used for a conductive communication vector can also be referred to herein as a timing scheme, as was noted above. In certain embodiments, a timing scheme can specify when conductive communication messages should be initiated relative to certain identifiable events, such as ventricular or atrial intrinsic events, ventricular or atrial paced events, asystole, diastole, and/or the like. For example, a first timing scheme can specify that a conductive communication message should be transmitted starting 50 msec after each ventricular sensed or paced event, a second timing scheme can specify that a conductive communication message should be transmitted starting 100 msec after each ventricular sensed or paced event, and a third timing scheme can specify that a conductive communication message should be transmitted starting 150 msec after each ventricular sensed or paced event.

In certain embodiments a default timing scheme, which can be considered a first timing scheme, can be used by a device that communicates with a second device using conductive communication and can continue to be used until the quality of the conductive communication falls below a corresponding threshold, at which point a second timing scheme can be used. Thereafter, the second timing scheme can be used until the quality of the conductive communication falls below the corresponding threshold, at which point a third timing scheme can be used, or alternatively, the device can revert back to using the first timing scheme. It is also possible that more than two or three different timing schemes can be used.

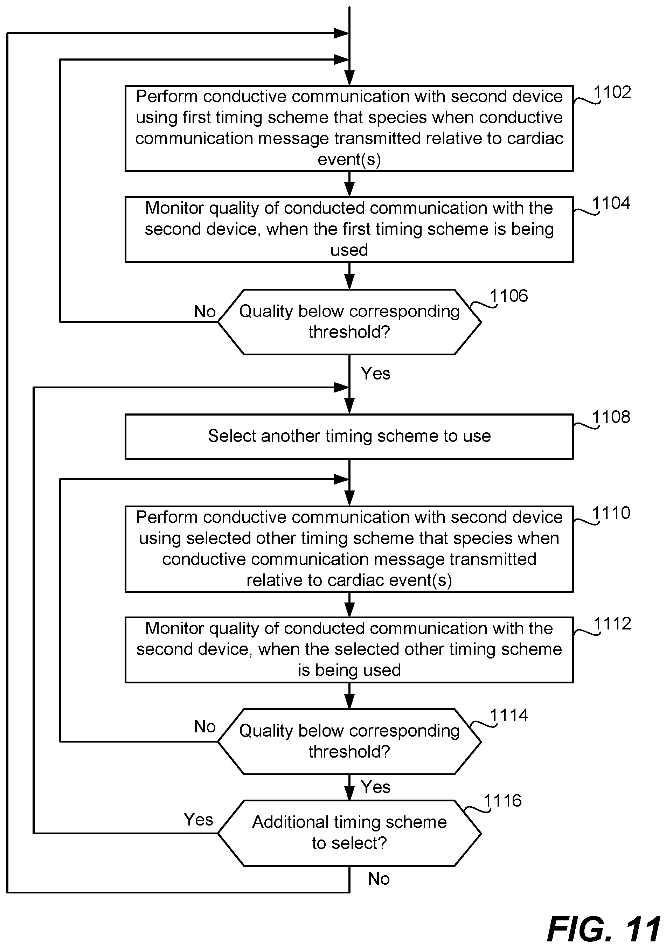

The high level flow diagram of FIG. 11 is used to summarize certain methods of changing between different timing schemes. Referring to FIG. 11, step 1102 involves a device performing conductive communication with a second device using a first timing scheme that specifies when a conductive communication message is transmitted relative to one or more cardiac events. The first timing scheme can be, for example, a default timing scheme. Step 1104 involves monitoring a quality of the conductive communication when using the first timing scheme, and step 1106 involves determining whether the quality is below a corresponding threshold. If the answer to the determination at step 1106 is Yes, meaning the quality threshold is exceeded, then flow returns to step 1102 and the device continues to use the first timing scheme. If the answer to the determination at step 1106 is Yes, meaning the quality is below the corresponding threshold, then flow goes to step 1108 where another timing scheme is selected for use.

Step 1110 involves the device performing conductive communication with the second device using the other selecting timing scheme (e.g., a second timing scheme that differs from the first timing scheme) that specifies when a conductive communication message is transmitted relative to one or more cardiac events. Step 1112 involves monitoring a quality of the conductive communication when using the selected other timing scheme, and step 1114 involves determining whether the quality is below a corresponding threshold. If there answer to the determination at step 1114 is No, then flow returns to step 1110 and the timing scheme selected at the most recent instance of step 1108 continues to be used. If the answer to the determination at step 1114 is Yes, then flow goes to step 1116.

At step 1116 there is a determination of whether there are additional timing schemes to select among. If the answer to the determination at step 1116 is Yes, then flow returns to step 1108 and another timing scheme is selected, and then used at the next instance of step 1110. If the answer to the determination at step 1116 is No, then flow returns to step 1102 and the device reverts to using the first timing scheme.

In certain embodiments, before changing from the first timing scheme to another (e.g., second) timing scheme, a device can first attempt to use different conductive communication vectors. More specifically, where the device includes and/or is communicatively coupled to at least three electrodes and can perform conductive communication using two or more different conductive communication vectors, the device may use the first timing scheme to perform conductive communication with the second device, using two or more of the plurality of different conductive communication vectors, before the changing to using the second timing scheme for performing the conductive communication with the second device. In certain such embodiments, the device changes to using the second timing scheme for performing the conductive communication with the second device in response to a quality metric being below the corresponding threshold when the first timing scheme is being used to perform the conducted communication with the second device, for each of the two or more of the plurality of different conductive communication vectors. In other words, in certain embodiments, a device changes its timing scheme only after multiple different conductive communication vectors are tried, and none of the vectors provide acceptable communication quality. Alternatively, different timing schemes can be tried before different conductive communication vectors are tried. In other words, in certain embodiments each timing scheme as part of a multi timing scheme protocol may be utilized, and the conductive communication vector that is used for the message transmissions is only changed after none of the timing schemes provided conductive communication quality that is acceptable.

In accordance with certain embodiments, if communication quality is below the corresponding threshold (when the first timing scheme is being used to perform the conductive communication with the second device), the transmitting device (which can be an IMD or an external device) evaluates whether or not noise detection has occurred during and/or within +/- X ms (where X is a programmable value) of a conductive communication transmission window of the first timing scheme. If noise has been detected within that time period (i.e., within the conductive communication transmission window of the first timing scheme), then the transmitting device (which can be an IMD or an external device) determines the time frame when noise had been detected, and defines a second timing scheme so that transmissions of conductive communication signals do not occur during the conductive communication transmission window used with the first timing scheme. In other words, the second timing scheme is devised to purposely avoid the detected noise. A goal of such an embodiment is to define a new timing scheme (e.g., the second timing scheme) in case of poor or unsuccessful conductive communication and use noise detection to define the new conductive communication transmission timing window for use with the second timing scheme.

In certain embodiments, when there is a change from a first conductive communication vector to a second conductive communication vector, the second conductive communication vector is approximately orthogonal to the first conductive communication vector. In other embodiments, an angle between the second conductive communication vector and the first conductive communication vector is approximately an odd integer multiple of 45 degrees. Other variations are also possible and within the scope of the embodiments described herein. Information about angles between different vectors can be determined during or following implantation of IMDs and can be stored in memory of IMDs and/or external devices, and thereafter accessed by the controllers thereof.

Where a timing scheme is relative to cardiac events, e.g., intrinsic or paced cardiac events, then a temporal spacing between the transmission of conductive communication message may remain substantially the same so long as the intrinsic or paced heart rate remains substantially the same. For example, assume that a first timing scheme specifies that a conductive communication message should be transmitted starting 50 msec after each ventricular sensed or paced event, and that the patient's heart rate is 60 beats per minute. This would result in a conductive communication message being transmitted once per second (i.e., once per 1000 msec), in which case the temporal spacing between when a conductive communication message and a next conductive communication message that is transmitted is one second (i.e., 1000 msec).

In accordance with certain embodiments, a device that includes and/or is communicatively coupled to at least three electrodes can receive a conductive communication signal (transmitted by another device) simultaneously using multiple different conductive communication vectors, and in certain such embodiments using each of at least three different conductive communication vectors. This enables the device to produce a respective bitstream, for each of the three (or more) different conductive communication vectors, to thereby produce three or more separate bitstreams. The device can then select or produce a valid bitstream based on the three or more separate bitstreams, determine message data included in and/or decoded from the valid bitstream, and then store and/or use the message data. Such message data can include information relating to a detected arrhythmia, EGM information, information related to remaining longevity of a device, and/or information related to errors or other problems with a device. Such message data can alternatively include instructions to reconfigure and/or reprogram a device. Other and/or additional types of information can be included within the such message data.

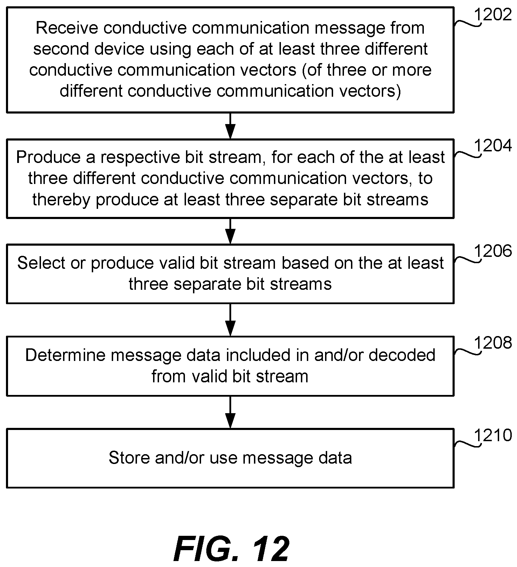

Methods that can be performed by such a device are described below with reference to the high level flow diagram of FIG. 12. In accordance with certain embodiments, the device that performs such a method can be an external device (e.g., 109) that is communicatively coupled to or comprises at least three skin electrodes (e.g., 115), and the second device (with which the external device communicates) can be an IMD. Alternatively, the device that performs such a method can be a non-vascular implantable cardioverter defibrillator (NV-ICD), and the second device comprises an intracardiac IMD. Other variations are also possible and within the scope of the embodiments described herein.

Referring to FIG. 12, step 1202 involves the device receiving a conductive communication message from the second device using each of at least three different conductive communication vectors of the three or more different conductive communication vectors. For example, referring briefly back to FIG. 7, where the device performing the method is the external device 109, a first conductive communication vector can include electrodes 115a and 115b, a second conductive communication vector can include electrodes 115a and 115c, and a third conductive communication vector can include electrodes 115b and 115c. A further conductive communication vector can include electrode 115a, and the electrodes 115b and 115c connected to one another. Still another conductive communication vector can include electrode 115b, and the electrodes 115a and 115c connected to one another. These are just a few examples which are not intended to be all encompassing. Referring briefly back to FIG. 8, where the device performing the method is the ICD 106, a first conductive communication vector can include electrodes 125a and 125b, a second conductive communication vector can include electrodes 125a and 125c, a third conductive communication vector can include electrodes 125b and 125c, etc. It is also possible to connect two (or more) of the electrodes 125 to one another. As noted above, when two or more electrodes (e.g., 125b and 125c) are connected to one another and thereby to a same node (e.g., to the same input node of the message amplifier/filter 740), such a configuration can effectively average or create a virtual vector which is between the two or more electrode locations, which enables sensing of a signal that is effectively an average of the signals detected at the two separate electrodes.

Referring again to FIG. 12, step 1204 involves producing a respective bitstream, for each of the at least three different conductive communication vectors, to thereby produce at least three separate bitstreams. Step 1206 involves selecting or producing a valid bitstream based on the at least three separate bitstreams. Step 1208 involves determining message data included in and/or decoded from the valid bitstream. Step 1210 involves storing and/or using the message data. For example, where the message data provides an instruction or command to the device that received the message, the device can perform the instruction or command. Such an instruction or command can be, e.g., a pacing rate adjustment, a transmission power level adjustment, etc. For another example, where the message data informs the device that received the message of a specific event (e.g., a pace or sensed cardiac event), the device that received the message can start a timer (e.g., an AV interval timer), determine whether an arrhythmia is detected, and/or the like. Message data can also provide an indication that an arrythmia has been detected, so that the device that received the message can respond according. Message data can also provide an indication of remaining battery longevity of the transmitting device, and/or the like. These are just a few examples of the types of information that can be included in message data transmitted from a first device to a second device, which examples are not intended to be all encompassing.

In certain embodiments, error detection is performed on each of the at least three separate bitstreams to thereby determine whether at least one of the at least three separate bitstreams is error-free. Such error detection can be performed, for example, using a check sum, but is not limited thereto. In certain such embodiments, when at least one of the at least three separate bitstreams is determined to be error-free, step 1206 is performed by selecting as the valid bitstream one bitstreams that is determined to be error-free. It is also noted that when one of the separate bitstreams is determined to be error-free, a controller can select the conductive communication vector, which was used to produce the error-free bitstream, as a preferred conductive communication vector for the device to use for performing further conductive communication with the second device.

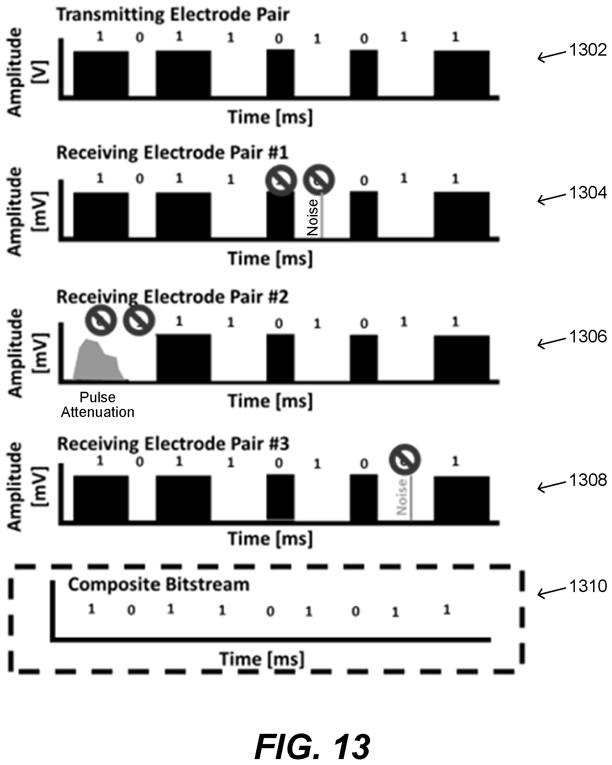

In certain embodiments, when none of the at least three separate bitstreams is determined to be error-free, step 1206 can involve producing a composite bitstream from the at least three separate bitstreams. In certain such embodiments, error detection can be performed on the composite bitstream to thereby determine whether the composite bitstream is error-free, and the composite bitstream can be used as the valid bitstream, if the composite bitstream is determined to be error-free. In accordance with certain embodiments, the composite bitstream is determined from the at least three separate bitstreams by identifying common bits in at least a majority of the at least three separate bitstreams. In other words, majority voting can be used to determine the composite bitstream. FIG. 13 illustrates how a composite bitstream 1310 that is the same as a transmitted bitstream 1302 can be generated by identifying common bits in at least a majority of the three separate bitstreams 1304, 1306 and 1308 that are produced by a receiving device. In such an embodiment, if a majority of bits (e.g., at least two out of three bits) in a same bit position in at least three (or more) bit streams are the same, it is assumed that the majority that are the same corresponds to a valid bit. For example, looking at the first (leftmost) bit position of the bitstreams 1304, 1306 and 1308, the bit value is 1 in the bitstream 1304, the bit value is 0 in the bitstream 1306, and the bit value is 1 on the bitstream 1308. Since for the first (leftmost) bit position, two out of three of the bit of the bitstreams 1304, 1306 and 1308 have the bit value of 1, it is determined that the bit value of the first (leftmost) bit position is 1, and a bit value of 1 is included in the first (leftmost) bit position in the composite bitstream 1310. Such a method mitigates effects of geometrically localized noise and pulse attenuation via assessment of bitstream agreeance across multiple receiving electrode pairs or combinations, wherein the different electrode pairs or combinations correspond to different conductive communication vectors.

Referring again to FIG. 12, in accordance with certain embodiments, step 1206 involves determining that at least two bitstreams of the at least three separate bitstreams match one another, and in response thereto, determining that the at least two bitstreams that match one another comprise the valid bitstream. For example, if two of the three bits streams are 101101011, and the third bitstream differs from 101101011, it is assumed that the differing bitstream has at least one error, and the valid bitstream is determined to be 101101011.

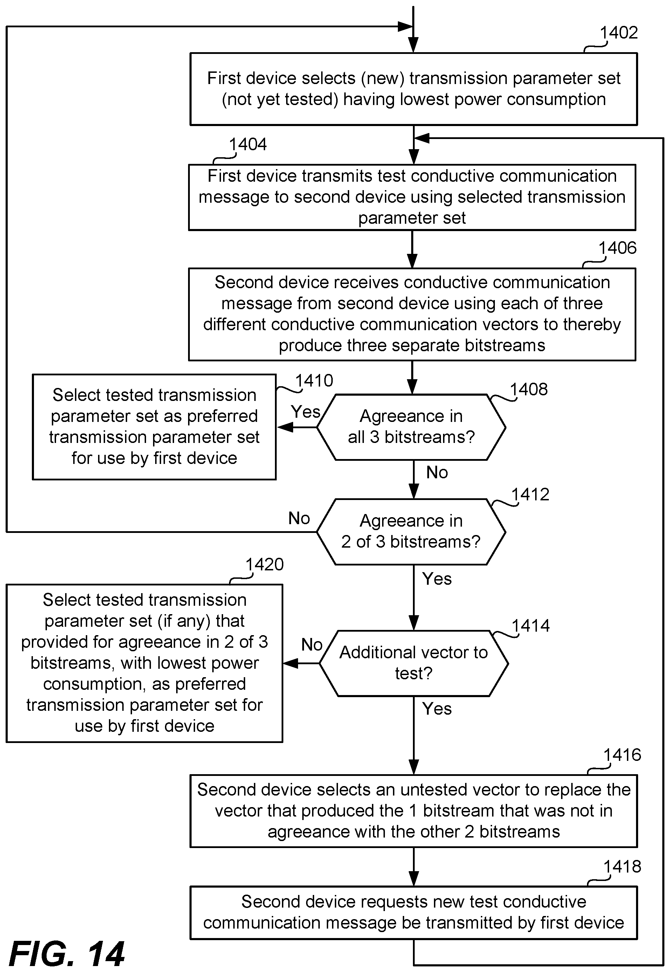

FIG. 14 is a high level flow diagram that is used to summarize certain methods for optimizing selecting of a transmission parameter set that is to be used by a first device for transmitting conductive communication messages to a second device, and optimizing receiving conductive communication vectors to be used by the second device for receiving conductive communication messages from the first device. The embodiments described with reference to FIG. 14 are especially useful where the first and second devices are both IMDs, and the second device includes and/or is communicatively coupled to at least three electrodes and can perform the conductive communication using a plurality of different conductive communication vectors. Conductive communication vectors are also sometimes referred to herein more succinctly as communication vectors, or even more succinctly as vectors.

Referring to FIG. 14, step 1402 involves the first device selecting a transmission parameter set, not yet tested, that has the lowest power consumption by the first device. The transmission parameter set can specify a binary data encoding protocol, pulse width, pulse amplitude, and/or data transmission frequency. Example data encoding protocols that can be used include, e.g., pulse width modulation (PWM), pulse amplitude modulation (PAM), pulse position modulation (PPM), pulse shift key (PSK), frequency shift key (PSK), amplitude shift key (ASK), quadrature amplitude modulation (QAM), but are not limited thereto.

Step 1404 involves the first device transmitting a test conductive communication message to the second device using the transmission parameter set selected at the most recent instance of step 1402. Between step 1402 and 1404, the first device may need to inform the second device, via another conductive communication message, that the first device is changing its encoding protocol, so that the second device knows how to decode the conductive communication messages it receives from the first device.

Step 1406 involves the second device receiving the test conductive communication message using each of three different conductive communication vectors to thereby produce three separate bit streams. Additional details of step 1406 can be appreciated from the above discussion of steps 1202 and 1204, and thus, need not be repeated.

Step 1408 involves the second device determining whether all three bitstreams are in agreeance, i.e., are the same. If the answer to the determination at step 1408 is Yes, then the second device provides an indication to the first device that all three bitstreams were in agreeance, and at step 1410 the first device selects the tested transmission parameter set as the preferred transmission parameter set that is to be used by the first device when transmitting conductive communication messages to the second device. This enables the first device to select a parameter set that provide for both low power consumption and acceptable conductive communication quality.

If the answer to the determination at step 1408 is No, then at step 1412 there is a determination of whether two-out-of-three of the bitstreams were in agreeance, i.e., were the same.

If the answer to the determination at step 1412 is No, which means none of the three bit streams were in agreeance with one another, then the second device informs the first device of such results and flow returns to step 1402 and a new transmission parameter set is selected for testing, at which point steps 1404, 1406, 1408, and potentially also step 1412 are repeated for the new transmission parameter set.

If the answer to the determination at step 1412 is Yes, then at step 1414 there is a determination of whether there is an additional (untested) receiving vector for the second device to test to replace the one receiving vector that produced the bitstream that was not in agreeance with the other two bitstreams produced by the other two receiving vectors. While not specifically shown in FIG. 14, if the answer to the determination at step 1412 is Yes, then the second device can also inform the first device via a conductive communication message that two-out-of-three bitstreams were in agreeance, so that the first device can save such information, e.g., to thereafter use at step 1420 if necessary.

If the answer to the determination at step 1414 is Yes, then at step 1416 the second device selects a new receiving vector to replace the one receiving vector that produced the bitstream that was not in agreeance with the other two bitstreams. Then at step 1418 the second device sends a conductive communication message to the first device to request that the first device send a new test conductive communication message to the first device, without changing that transmission parameter set. Steps 1404, 1406, etc., are then repeated.

If at some point the answer to the determination at step 1414 is No, then the second device provides an indication to the first device that having two-out-of-three bitstreams to be in agreeance was the best result achieved. Then at step 1420, the first device selects as the preferred transmission parameter set, the parameter set that provided for agreeance in two-out-of-three bitstreams, and provided for the lowest power consumption by the first device if multiple parameter sets provided for agreeance in two-out-of-three bitstreams.

The methods summarized with reference to FIG. 14, which can be performed by the controllers of the first and second devices, can be used by the first and second devices to select the transmission parameter set that provides for low power consumption (by the first device) and acceptable conductive communication quality.

FIGS. 15A and 15B show an example of how the position of an LP 102, relative to a case electrode 125a of an NV-ICD 106 and electrodes 125b, 125c, 125d, and 125e that are located on a lead 103 that is connected to the NV-ICD 106, can change depending upon the posture of the patient in which the LP and NV-ICD are implanted. In FIG. 15A the patient is in a left lateral posture, and in FIG. 15B the patient is in a right lateral posture. As can be appreciated from FIGS. 15A and 15B, the relative position of the LP 102 to the various electrodes 125 changes at least in part depending upon the posture of the patient. This is in part due to the heart moving laterally due to the force of gravity in a direction the depends on the posture of the patient. This can result in the preferred conductive communication vector being different for different postures, as was explained above, e.g., with reference to FIG. 9 and 10. For a specific example, the preferred conductive communication vector can include the proximal ring electrode 125c and the distal coil electrode 125d when the patient is in the left lateral posture, and the preferred conductive communication vector can include the distal ring (or tip) electrode 125b and the proximal ring electrode 125c when the patient is in the right lateral posture.

An aspect of the embodiments relates to a method for use by a first device that is configured to communicate with a second device using conductive communication, wherein the first device includes and/or is communicatively coupled to at least three electrodes and can perform the conductive communication using a plurality of different conductive communication vectors, wherein each of the plurality of different conductive communication vectors comprises a different combination of the at least three electrodes, wherein the second device includes and/or is communicatively coupled to at least two additional electrodes that can be used to perform the conductive communication, and wherein at least one of the first or second devices comprises an implantable medical device (IMD) implanted in a patient, the method for use by the first device comprising: obtaining information indicative of at least one of a physical or physiologic state of the patient; identifying which one of the plurality of different conductive communication vectors is a preferred conductive communication vector for communicating with the second device, based on the information indicative of the at least one of the physical or physiologic state of the patient; and performing the conductive communication with the second device using the preferred conductive communication vector.

In an embodiment, the obtaining information indicative of the at least one of the physical or physiologic state of the patient comprises obtaining information indicative of at least one of a posture or activity level of the patient; and the identifying which one of the plurality of different conductive communication vectors is the preferred conductive communication vector for communicating with the second device, is performed based the information indicative of the at least one of the posture or activity level of the patient.

In an embodiment, the information indicative of the at least one of the posture or activity level of the patient comprises, or is based on, one or more signals output from at least one of an accelerometer or a temperature sensor.

In an embodiment, the information indicative of the at least one of the physical or physiologic state of the patient comprises, or is based on, at least one of an impedance measurement, an electrocardiogram (ECG), or an electrogram (EGM) measurement.

In an embodiment, the first device comprises an external device; the at least three electrodes, that the first device includes and/or is communicatively coupled to, comprise at least three skin electrodes; and the second device comprises the IMD.

In an embodiment, the first device comprises a non-vascular implantable cardioverter defibrillator (NV-ICD), and the second device comprises an intracardiac IMD.

In an embodiment, the first device is also configured to communicate with a third device that includes and/or is communicatively coupled to at least two further electrodes that can be used to perform further conductive communication, wherein the third device comprises a further IMD implanted in the patient, and wherein the method for use by the first device further comprises: identifying which one of the plurality of different conductive communication vectors is a further preferred conductive communication vector for communicating with the third device, based on the information indicative of the at least one of the physical or physiologic state of the patient; and performing the further conductive communication with the third device using the further preferred conductive communication vector.

wherein an embodiment, the preferred conductive communication vector is used for both transmitting conductive communication signals to the second device and receiving conductive communication signals from the second device.

In an embodiment, the preferred conductive communication vector is used for one of transmitting conductive communication signals to the second device or receiving conductive communication signals from the second device; a further preferred conductive communication vector is used for the other one of transmitting conductive communication signals to the second device or receiving conductive communication signals from the second device; and the method for use by the first device further comprises identifying the further preferred conductive communication vector, and performing the conductive communication with the second device also using the further preferred conductive communication vector.

In an embodiment, the obtaining, the identifying and the performing are repeated from time to time such that the preferred conductive communication vector may change in response to the at least one of the physical or physiologic state of the patient changing.

In an embodiment, the at least one of the physical or physiologic state of the patient comprises at least one state of at least one cyclical physiologic cycle that includes at least one of a cardiac cycle, a respiratory cycle, or another type of cyclical physiologic cycle; and the obtaining, the identifying and the performing are performed at least twice during a same cyclical physiologic cycle such that the preferred conductive communication vector changes within the same cyclical physiologic cycle, resulting in at least two of the plurality of different conductive communication vectors being used to perform the conductive communication with the second device during the same cyclical physiologic cycle.

This aspect of the embodiments also relate to a device configured to communicate with a second device using conductive communication, wherein at least one of the device or the second device comprises an implantable medical device (IMD) configured to be implanted in a patient, the device comprising: conductive communication circuitry; switches between the conductive communication circuitry and at least three electrodes that are part of or communicatively coupled to the device; a controller configured to control the switches to thereby enable the device to perform the conductive communication using a plurality of different conductive communication vectors, wherein each of the plurality of different conductive communication vectors comprises a different combination of the at least three electrodes; the controller further configured to obtain information indicative of at least one of a physical or physiologic state of the patient, identify which one of the plurality of different conductive communication vectors is a preferred conductive communication vector for communicating with the second device, based on the information indicative of the at least one of the physical or physiologic state of the patient, and control the switches to cause the conductive communication with the second device to be performed using the preferred conductive communication vector.

In an embodiment, the information indicative of the at least one of the physical or physiologic state of the patient comprises information indicative of at least one of a posture or activity level of the patient; and the controller is configured to identify which one of the plurality of different conductive communication vectors is the preferred conductive communication vector for communicating with the second device, based the information indicative of the at least one of the posture or activity level of the patient.

In an embodiment, the device includes at least one of an accelerometer or temperature sensor, and the information indicative of at least one of posture or activity level of the patient comprises, or is based on, one or more signals output from at least one of the accelerometer or temperature sensor.

In an embodiment, the information indicative of the at least one of the physical or physiologic state of the patient comprises, or is based on, at least one of an impedance measurement, an electrocardiogram (ECG) measurement, or an electrogram (EGM) measurement.

In an embodiment the device comprises an external device; the at least three electrodes, that the device includes and/or is communicatively coupled to, comprise at least three skin electrodes; and the second device comprises the IMD.

In an embodiment, the device comprises a non-vascular implantable cardioverter defibrillator (NV-ICD), and the second device comprises an intracardiac IMD.

In an embodiment, the device is also configured to communicate with a third device that includes and/or is communicatively coupled to at least two further electrodes that can be used to perform further conductive communication, and wherein the third device comprises a further IMD configured to be implanted in a patient, the controller further configured to: identify which one of the plurality of different conductive communication vectors is a further preferred conductive communication vector for communicating with the third device, based on the information indicative of the at least one of the physical or physiologic state of the patient; and control the switches to cause the further conductive communication with the third device to be performed using the further preferred conductive communication vector.

In an embodiment, the conductive communication circuitry comprises a conductive communication transmitter and a conductive communication receiver; and the preferred conductive communication vector is used by the conductive communication transmitter for transmitting conductive communication signals to the second device, as well as being used by the conductive communication receiver for receiving conductive communication signals from the second device.

In an embodiment, the conductive communication circuitry comprises a conductive communication transmitter and a conductive communication receiver; the preferred conductive communication vector is used for one of transmitting conductive communication signals to the second device or receiving conductive communication signals from the second device; a further preferred conductive communication vector is used for the other one of transmitting conductive communication signals to the second device or receiving conductive communication signals from the second device; and the controller is further configured to identify the further preferred conductive communication vector, and control the switches to cause conductive communication with the second device also using the further preferred conductive communication vector.

In an embodiment, the controller is configured to: identify a new preferred conductive communication vector when the at least one of the physical or physiologic state of the patient changes; and control the switches to cause the conductive communication with the second device to be performed using the new preferred conductive communication vector.

In an embodiment, the at least one of the physical or physiologic state of the patient comprises at least one state of at least one cyclical physiologic cycle that includes at least one of a cardiac cycle, a respiratory cycle, or another type of cyclical physiologic cycle; and the controller is configured to identify the preferred conductive communication vector at least twice during a same cyclical physiologic cycle such that the preferred conductive communication vector changes within the same cyclical physiologic cycle; and control the switches to cause the conductive communication with the second device to be performed using the least two of the plurality of different conductive communication vectors during the same cyclical physiologic cycle.

Another aspect of the embodiments relates to a method for use by a first device that is configured to communicate with a second device using conductive communication, wherein the first device includes and/or is communicatively coupled to three or more electrodes and can receive a conductive communication message using three or more different conductive communication vectors, wherein each of the three or more different conductive communication vectors comprises a different combination of the three or more electrodes, wherein the second device includes and/or is communicatively coupled to at least two additional electrodes that can be used to transmit the conductive communication message, and wherein at least one of the first and second devices comprises an implantable medical device (IMD) implanted in a patient, the method for use by the first device comprising: receiving the conductive communication message from the second device using each of at least three different conductive communication vectors of the three or more different conductive communication vectors; producing a respective bitstream, for each of the at least three different conductive communication vectors, to thereby produce at least three separate bitstreams; and selecting or producing a valid bitstream based on the at least three separate bitstreams; determining message data included in and/or decoded from the valid bitstream; and at least one of storing or using the message data.

In an embodiment, the method also comprises performing error detection on each of the at least three separate bitstreams to thereby determine whether at least one of the at least three separate bitstreams is error-free.

In an embodiment, when at least one of the at least three separate bitstreams is determined to be error-free, the selecting or producing the valid bitstream comprises selecting as the valid bitstream at least one of the at least three separate bitstreams that is determined to be error-free.

In an embodiment, when one of the at least three separate bitstreams is determined to be error-free, the conductive communication vector used to produce the error-free bitstream is selected as a preferred conductive communication vector for the first device to use to receive a further conductive communication message from the second device.

In an embodiment, when none of the at least three separate bitstreams is determined to be error-free, the selecting or producing the valid bitstream comprises: producing a composite bitstream from the at least three separate bitstreams.

In an embodiment, the method further comprises performing error detection on the composite bitstream to thereby determine whether the composite bitstream is error-free; and using the composite bitstream as the valid bitstream in response to the composite bitstream being determined to be error-free.

In an embodiment, the producing the composite bitstream from the at least three separate bitstreams includes identifying common bits in at least a majority of the at least three separate bitstreams.

In an embodiment, the methos further comprises determining that at least two bitstreams of the at least three separate bitstreams match one another, and in response thereto, determining that the at least two bitstreams that match one another comprise the valid bitstream.

In an embodiment, the first device comprises an external device; the three or more electrodes, that the first device includes and/or is communicatively coupled to, comprise three or more skin electrodes; and the second device comprises an IMD.

In an embodiment, the first device comprises a non-vascular implantable cardioverter defibrillator (NV-ICD), and the second device comprises an intracardiac IMD.

This aspect of the embodiments also relates to a device configured to communicate with a second device using conductive communication, wherein at least one of the device or the second device comprises an implantable medical device (IMD) configured to be implanted in a patient, the device comprising: conductive communication circuitry; switches between the conductive communication circuitry and at least three electrodes that are part of or communicatively coupled to the device, wherein the switches enable three or more different conductive communication vectors to be electrically coupled to the conductive communication circuitry; and a controller configured to control the switches to thereby enable the device to receive a conductive communication message from the second device using each of at least three different conductive communication vectors of the three or more different conductive communication vectors; wherein the conductive communication circuitry is configured to produce a respective bitstream, for each of the at least three different conductive communication vectors, to thereby produce at least three separate bitstreams; and wherein the controller is further configured to receive the at least three separate bitstreams from the conductive communication circuitry, select or produce a valid bitstream based on the at least three separate bitstreams, determine message data included in and/or decoded from the valid bitstream, and at least one of store or use the message data.

In an embodiment, the controller is further configured to: perform error detection on each of the at least three separate bitstreams to thereby determine whether at least one of the at least three separate bitstreams is error-free.

In an embodiment, when the controller determines that at least one of the at least three separate bitstreams is error-free, the controller is configured to select as the valid bitstream at least one of the at least three separate bitstreams that is determined to be error-free.

In an embodiment, when the controller determines that one of the at least three separate bitstreams is determined to be error-free, the controller is configured to select the conductive communication vector that was used to produce the error-free bitstream as a preferred conductive communication vector for the device to use to receive a further conductive communication message from the second device.

In an embodiment, when the controller determines that none of the at least three separate bitstreams is determined to be error-free, the controller is configured to produce the valid bitstream by producing a composite bitstream from the at least three separate bitstreams.

In an embodiment, the controller is configured to: perform error detection on the composite bitstream to thereby determine whether the composite bitstream is error-free; and use the composite bitstream as the valid bitstream in response to the composite bitstream being determined to be error-free.

In an embodiment, the controller is configured to produce the composite bitstream from the at least three separate bitstreams by identifying common bits in at least a majority of the at least three separate bitstreams.

In an embodiment, the controller is configured to: determine when at least two bitstreams of the at least three separate bitstreams match one another, and in response thereto, determine that the at least two bitstreams that match one another comprise the valid bitstream.

In an embodiment, the device comprises an external device; the at least three electrodes, that the device includes and/or is communicatively coupled to, comprise at least three skin electrodes; and the second device comprises an IMD.

In an embodiment, the device comprises a non-vascular implantable cardioverter defibrillator (NV-ICD), and the second device comprises an intracardiac IMD.

A further aspect of the embodiments relates to a method for use by a first device that is configured to communicate with a second device using conductive communication, wherein at least one of the first or second devices comprises an implantable medical device (IMD) implanted in a patient, the method for use by the first device comprising: performing the conductive communication with the second device using a first timing scheme that species when conductive communication pulses are transmitted relative to one or more cardiac events; monitoring a quality metric of the conductive communication with the second device, when the first timing scheme is being used to perform the conductive communication with the second device; detecting the quality metric being below a corresponding threshold, when the first timing scheme is being used to perform the conductive communication with the second device; and in response to detecting the quality metric being below the corresponding threshold, when the first timing scheme is being used to perform the conductive communication with the second device, changing to using a second timing scheme for performing the conductive communication with the second device; wherein the second timing scheme differs from the first timing scheme and specifies when conductive communication pulses are transmitted relative to one or more cardiac events.

In an embodiment, the method further comprises after the changing to using the second timing scheme, monitoring a quality metric of the conductive communication with the second device when using the second timing scheme for performing the conductive communication with the second device; detecting the quality metric being below the corresponding threshold, when using the second timing scheme for performing the conductive communication with the second device; and in response to detecting the quality metric being below the corresponding threshold, when using the second timing scheme for performing the conductive communication with the second device, changing to using a third timing scheme for performing the conductive communication with the second device; wherein the third timing scheme differs from the first and second timing schemes and specifies when conductive communication pulses are transmitted relative to one or more cardiac events.

In an embodiment, the first device includes and/or is communicatively coupled to at least three electrodes and can perform the conductive communication using a plurality of different conductive communication vectors; each of the plurality of different conductive communication vectors comprises a different combination of the at least three electrodes; the performing the conductive communication with the second device using the first timing scheme is performed using two or more of the plurality of different conductive communication vectors, before the changing to using the second timing scheme for performing the conductive communication with the second device; and the changing to using the second timing scheme for performing the conductive communication with the second device occurs in response to the quality metric being below the corresponding threshold when the first timing scheme is being used to perform the conductive communication with the second device, for each of the two or more of the plurality of different conductive communication vectors.

In an embodiment, there is a temporal spacing between when a conductive communication message and a next conductive communication message is transmitted from the first device to the second device, wherein the temporal spacing affects how frequent conductive communication messages are transmitted from the first device to the second device, and the method further comprising: after the changing to using the second timing scheme, monitoring a quality metric of the conductive communication with the second device when using the second timing scheme for performing the conductive communication with the second device; detecting the quality metric being below the corresponding threshold, when using the second timing scheme for performing the conductive communication with the second device; and in response to detecting the quality metric being below the corresponding threshold, when using the second timing scheme for performing the conductive communication with the second device, changing the temporal spacing.

In an embodiment, the first device includes and/or is communicatively coupled to at least three electrodes and can perform the conductive communication using a plurality of different conductive communication vectors; each of the plurality of different conductive communication vectors comprises a different combination of the at least three electrodes; after the changing to using the second timing scheme for performing the conductive communication with the second device, monitoring a quality metric of the conductive communication with the second device, when the second timing scheme is being used to perform the conductive communication with the second device; detecting the quality metric being below the corresponding threshold, when the second timing scheme is being used to perform the conductive communication with the second device; and changing from using a first conductive communication vector of the plurality of different conductive communication vectors to using a second conductive communication vector of the plurality of different conductive communication vectors for performing the conductive communication with the second device, in response to the quality metric being below the corresponding threshold when using each of the first and second timing schemes to perform the conductive communication with the second device using the first conductive communication vector.

In an embodiment, there is a temporal spacing between when one conductive communication message and a next conductive communication message is transmitted from the first device to the second device, wherein the temporal spacing affects how frequent conductive communication messages are transmitted from the first device to the second device, and the method further comprising: after the changing to using the second conductive communication vector, monitoring a quality metric of the conductive communication with the second device when using the second conductive communication vector for performing the conductive communication with the second device; and detecting the quality metric being below the corresponding threshold, when using the second conductive communication vector for performing the conductive communication with the second device; and in response to detecting the quality metric being below the corresponding threshold, when using the second conductive communication vector for performing the conductive communication with the second device, changing the temporal spacing.

In an embodiment, one of the first and second devices comprises an IMD; and the other one of the first and second devices comprises an external device.

In an embodiment, each of the first and second devices comprises a respective IMD.

In an embodiment, the first device comprises a non-vascular implantable cardioverter defibrillator (NV-ICD), and the second device comprises an intracardiac IMD.

This aspect of the embodiments also relates to a device configured to communicate with a second device using conductive communication, wherein at least one of the device or the second device comprises an implantable medical device (IMD) configured to be implanted in a patient, the device comprising: conductive communication circuitry; a controller configured to control the conductive communication circuitry to perform the conductive communication with the second device using a first timing scheme that specifies when conductive communication pulses are transmitted relative to one or more cardiac events, monitor a quality metric of the conductive communication with the second device, when the first timing scheme is being used to perform the conductive communication with the second device, detect the quality metric being below a corresponding threshold, when the first timing scheme is being used to perform the conductive communication with the second device, and in response to detecting the quality metric being below the corresponding threshold, when the first timing scheme is being used to perform the conductive communication with the second device, change to using a second timing scheme to perform the conductive communication with the second device; wherein the second timing scheme differs from the first timing scheme and specifies when conductive communication pulses are transmitted relative to one or more cardiac events.

In an embodiment, the controller is further configured to control the conductive communication circuitry to: after the changing to using the second timing scheme, monitor a quality metric of the conductive communication with the second device when using the second timing scheme for performing the conductive communication with the second device; detect the quality metric being below the corresponding threshold, when using the second timing scheme for performing the conductive communication with the second device; and in response to detecting the quality metric being below the corresponding threshold, when using the second timing scheme for performing the conductive communication with the second device, change to using a third timing scheme for performing the conductive communication with the second device; wherein the third timing scheme differs from the first and second timing schemes and specifies when conductive communication pulses are transmitted relative to one or more cardiac events.

In an embodiment, the device includes and/or is communicatively coupled to at least three electrodes and can perform the conductive communication using a plurality of different conductive communication vectors; each of the plurality of different conductive communication vectors comprises a different combination of the at least three electrodes; the controller is further configured to control the conductive communication circuitry to use two or more of the plurality of different conductive communication vectors to perform the conductive communication with the second device using the first timing scheme; and change to using the second timing scheme to perform the conductive communication with the second device, in response to the quality metric being below the corresponding threshold when the first timing scheme is being used to perform the conductive communication with the second device for each of the two or more of the plurality of different conductive communication vectors.

In an embodiment, there is a temporal spacing between when a conductive communication message and a next conductive communication message is transmitted from the device to the second device, wherein the temporal spacing affects how frequent conductive communication messages are transmitted from the device to the second device, and wherein the controller is further configured to control the conductive communication circuitry to after the changing to using the second timing scheme, monitor a quality metric of the conductive communication with the second device when using the second timing scheme to perform the conductive communication with the second device; detect the quality metric being below the corresponding threshold, when using the second timing scheme to perform the conductive communication with the second device; and change the temporal spacing in response to detecting the quality metric being below the corresponding threshold when using the second timing scheme for performing the conductive communication with the second device.

In an embodiment, the device includes and/or is communicatively coupled to at least three electrodes and can perform the conductive communication using a plurality of different conductive communication vectors; each of the plurality of different conductive communication vectors comprises a different combination of the at least three electrodes; the controller is further configured to control the conductive communication circuitry to monitor a quality metric of the conductive communication with the second device when the second timing scheme is being used to perform the conductive communication with the second device, after the change to using the second timing scheme for performing the conductive communication with the second device; detect the quality metric being below the corresponding threshold, when the second timing scheme is being used to perform the conductive communication with the second device; and change from using a first conductive communication vector of the plurality of different conductive communication vectors to using a second conductive communication vector of the plurality of different conductive communication vectors for performing the conductive communication with the second device, in response to the quality metric being below the corresponding threshold when using each of the first and second timing schemes to perform the conductive communication with the second device using the first conductive communication vector.

In an embodiment, there is a temporal spacing between when one conductive communication message and a next conductive communication message is transmitted from the device to the second device, wherein the temporal spacing affects how frequent conductive communication messages are transmitted from the device to the second device, and the controller is further configured to control the conductive communication circuitry to monitor a quality metric of the conductive communication with the second device when using the second conductive communication vector to perform the conductive communication with the second device, after the change to using the second conductive communication vector; detect the quality metric being below the corresponding threshold, when using the second conductive communication vector to perform the conductive communication with the second device; and change the temporal spacing in response to detecting the quality metric being below the corresponding threshold when using the second conductive communication vector for performing the conductive communication with the second device.

In an embodiment, the device comprises an external device; and the second device comprises an IMD.

In an embodiment, each of the device and the second device comprises a respective IMD.

In an embodiment, the device comprises a non-vascular implantable cardioverter defibrillator (NV-ICD), and the second device comprises an intracardiac IMD.

The description of the various embodiments as provided above and in connection with the drawings also apply to the various aspects and embodiments thereof disclosed herein.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope and spirit of the claimed invention. For example, it would be possible to combine or separate some of the steps shown in the various flow diagrams. It would also be possible to just perform a subset of the steps shown in the various flow diagrams. For another example, it is possible to change the boundaries of some of the block diagrams and flow diagrams. It would also be possible, and within the scope of the embodiments described herein, to change the order of some of the steps in the various flow diagram, such as in the flow diagrams of FIGS. 9-12 and 14.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A device (102a, 102b, 104, 106, 109) configured to communicate with a second device (102a, 102b, 104, 106, 109) using conductive communication, wherein at least one of the device or the second device comprises an implantable medical device, IMD, (102a, 102b, 104, 106) configured to be implanted in a patient, the device comprising:
conductive communication circuitry (116, 120, 752); and
a controller (112, 712) configured to:
control the conductive communication circuitry (116, 120, 752) to perform the conductive communication with the second device (102a, 102b, 104, 106, 109) using a first timing scheme that specifies when conductive communication pulses are transmitted relative to one or more cardiac events;
monitor a quality metric of the conductive communication with the second device (102a, 102b, 104, 106, 109), when the first timing scheme is being used to perform the conductive communication with the second device (102a, 102b, 104, 106, 109);
detect the quality metric being below a corresponding threshold, when the first timing scheme is being used to perform the conductive communication with the second device (102a, 102b, 104, 106, 109); and
in response to detecting the quality metric being below the corresponding threshold, when the first timing scheme is being used to perform the conductive communication with the second device, change to using a second timing scheme to perform the conductive communication with the second device (102a, 102b, 104, 106, 109);
wherein the second timing scheme differs from the first timing scheme and specifies when conductive communication pulses are transmitted relative to one or more cardiac events.

2. The device (102a, 102b, 104, 106, 109) of claim 1, wherein the controller (112, 712) is further configured to control the conductive communication circuitry (116, 120, 752) to:
after the changing to using the second timing scheme, monitor a quality metric of the conductive communication with the second device (102a, 102b, 104, 106, 109) when using the second timing scheme for performing the conductive communication with the second device (102a, 102b, 104, 106, 109);
detect the quality metric being below the corresponding threshold, when using the second timing scheme for performing the conductive communication with the second device (102a, 102b, 104, 106, 109); and
in response to detecting the quality metric being below the corresponding threshold, when using the second timing scheme for performing the conductive communication with the second device (102a, 102b, 104, 106, 109), change to using a third timing scheme for performing the conductive communication with the second device (102a, 102b, 104, 106, 109);
wherein the third timing scheme differs from the first and second timing schemes and specifies when conductive communication pulses are transmitted relative to one or more cardiac events.

3. The device (102a, 102b, 104, 106, 109) of any one of claims 1 or 2, wherein:
the device (102a, 102b, 104, 106, 109) includes and/or is communicatively coupled to at least three electrodes (108, 115, 125) and can perform the conductive communication using a plurality of different conductive communication vectors;
each of the plurality of different conductive communication vectors comprises a different combination of the at least three electrodes (108, 115, 125); and
the controller (112, 712) is further configured to:
control the conductive communication circuitry (116, 120, 752) to use two or more of the plurality of different conductive communication vectors to perform the conductive communication with the second device (102a, 102b, 104, 106, 109) using the first timing scheme; and
change to using the second timing scheme to perform the conductive communication with the second device (102a, 102b, 104, 106, 109), in response to the quality metric being below the corresponding threshold when the first timing scheme is being used to perform the conductive communication with the second device (102a, 102b, 104, 106, 109) for each of the two or more of the plurality of different conductive communication vectors.

4. The device (102a, 102b, 104, 106, 109) of any one of claims 1 through 3, wherein there is a temporal spacing between when a conductive communication message and a next conductive communication message is transmitted from the device (102a, 102b, 104, 106, 109) to the second device (102a, 102b, 104, 106, 109), wherein the temporal spacing affects how frequent conductive communication messages are transmitted from the device to the second device (102a, 102b, 104, 106, 109), and wherein the controller (112, 712) is further configured to:
control the conductive communication circuitry (116, 120, 752) to after the changing to using the second timing scheme, monitor a quality metric of the conductive communication with the second device (102a, 102b, 104, 106, 109) when using the second timing scheme to perform the conductive communication with the second device (102a, 102b, 104, 106, 109);
detect the quality metric being below the corresponding threshold, when using the second timing scheme to perform the conductive communication with the second device (102a, 102b, 104, 106, 109); and
change the temporal spacing in response to detecting the quality metric being below the corresponding threshold when using the second timing scheme for performing the conductive communication with the second device (102a, 102b, 104, 106, 109).

5. The device (102a, 102b, 104, 106, 109) of any one of claims 1 through 4, wherein:
the device (102a, 102b, 104, 106, 109) includes and/or is communicatively coupled to at least three electrodes (108, 115, 125) and can perform the conductive communication using a plurality of different conductive communication vectors;
each of the plurality of different conductive communication vectors comprises a different combination of the at least three electrodes (108, 115, 125);
the controller (112, 712) is further configured to:
control the conductive communication circuitry (116, 120, 752) to monitor a quality metric of the conductive communication with the second device (102a, 102b, 104, 106, 109) when the second timing scheme is being used to perform the conductive communication with the second device (102a, 102b, 104, 106, 109), after the change to using the second timing scheme for performing the conductive communication with the second device (102a, 102b, 104, 106, 109);
detect the quality metric being below the corresponding threshold, when the second timing scheme is being used to perform the conductive communication with the second device (102a, 102b, 104, 106, 109); and
change from using a first conductive communication vector of the plurality of different conductive communication vectors to using a second conductive communication vector of the plurality of different conductive communication vectors for performing the conductive communication with the second device (102a, 102b, 104, 106, 109), in response to the quality metric being below the corresponding threshold when using each of the first and second timing schemes to perform the conductive communication with the second device (102a, 102b, 104, 106, 109) using the first conductive communication vector.

6. The device (102a, 102b, 104, 106, 109) of any one of claims 1 through 5, wherein there is a temporal spacing between when one conductive communication message and a next conductive communication message is transmitted from the device (102a, 102b, 104, 106, 109) to the second device (102a, 102b, 104, 106, 109), wherein the temporal spacing affects how frequent conductive communication messages are transmitted from the device (102a, 102b, 104, 106, 109) to the second device (102a, 102b, 104, 106, 109), and the controller (112, 712) is further configured to:
control the conductive communication circuitry (116, 120, 752) to monitor a quality metric of the conductive communication with the second device (102a, 102b, 104, 106, 109) when using the second conductive communication vector to perform the conductive communication with the second device (102a, 102b, 104, 106, 109), after the change to using the second conductive communication vector;
detect the quality metric being below the corresponding threshold, when using the second conductive communication vector to perform the conductive communication with the second device (102a, 102b, 104, 106, 109); and
change the temporal spacing in response to detecting the quality metric being below the corresponding threshold when using the second conductive communication vector for performing the conductive communication with the second device (102a, 102b, 104, 106, 109).

7. The device (102a, 102b, 104, 106, 109) of any one of claims 1 through 6, wherein:
the device (102a, 102b, 104, 106, 109) comprises an external device (109) and the second device (102a, 102b, 104, 106, 109) comprises an IMD (102a, 102b, 104, 106); or
each of the device (102a, 102b, 104, 106, 109) and the second device (102a, 102b, 104, 106, 109) comprises a respective IMD (102a, 102b, 104, 106).

8. A system (100) including the device (102a, 102b, 104, 106, 109) of any one of claims 1 through 7, and also including the second device (102a, 102b, 104, 106, 109).

9. A method for use by a first device (102a, 102b, 104, 106, 109) that is configured to communicate with a second device (102a, 102b, 104, 106, 109) using conductive communication, wherein at least one of the first or second devices (102a, 102b, 104, 106, 109) comprises an implantable medical device, IMD, (102a, 102b, 104, 106) configured to be implanted in a patient, the method for use by the first device (102a, 102b, 104, 106, 109) comprising:
performing the conductive communication with the second device (102a, 102b, 104, 106, 109) using a first timing scheme that specifies when conductive communication pulses are transmitted relative to one or more cardiac events;
monitoring a quality metric of the conductive communication with the second device (102a, 102b, 104, 106, 109), when the first timing scheme is being used to perform the conductive communication with the second device (102a, 102b, 104, 106, 109);
detecting the quality metric being below a corresponding threshold, when the first timing scheme is being used to perform the conductive communication with the second device (102a, 102b, 104, 106, 109); and
in response to detecting the quality metric being below the corresponding threshold, when the first timing scheme is being used to perform the conductive communication with the second device (102a, 102b, 104, 106, 109), changing to using a second timing scheme for performing the conductive communication with the second device (102a, 102b, 104, 106, 109);
wherein the second timing scheme differs from the first timing scheme and specifies when conductive communication pulses are transmitted relative to one or more cardiac events.

10. The method of claim 9, further comprising:
after the changing to using the second timing scheme, monitoring a quality metric of the conductive communication with the second device when using the second timing scheme for performing the conductive communication with the second device (102a, 102b, 104, 106, 109);
detecting the quality metric being below the corresponding threshold, when using the second timing scheme for performing the conductive communication with the second device (102a, 102b, 104, 106, 109); and
in response to detecting the quality metric being below the corresponding threshold, when using the second timing scheme for performing the conductive communication with the second device (102a, 102b, 104, 106, 109), changing to using a third timing scheme for performing the conductive communication with the second device (102a, 102b, 104, 106, 109);
wherein the third timing scheme differs from the first and second timing schemes and specifies when conductive communication pulses are transmitted relative to one or more cardiac events.

11. The method of any one of claims 9 or 10, wherein:
the first device (102a, 102b, 104, 106, 109) includes and/or is communicatively coupled to at least three electrodes (108, 115, 125) and can perform the conductive communication using a plurality of different conductive communication vectors;
each of the plurality of different conductive communication vectors comprises a different combination of the at least three electrodes (108, 115, 125);
the performing the conductive communication with the second device (102a, 102b, 104, 106, 109) using the first timing scheme is performed using two or more of the plurality of different conductive communication vectors, before the changing to using the second timing scheme for performing the conductive communication with the second device (102a, 102b, 104, 106, 109); and
the changing to using the second timing scheme for performing the conductive communication with the second device (102a, 102b, 104, 106, 109) occurs in response to the quality metric being below the corresponding threshold when the first timing scheme is being used to perform the conductive communication with the second device (102a, 102b, 104, 106, 109), for each of the two or more of the plurality of different conductive communication vectors.

12. The method of any one of claims 9 through 11, wherein there is a temporal spacing between when a conductive communication message and a next conductive communication message is transmitted from the first device (102a, 102b, 104, 106, 109) to the second device (102a, 102b, 104, 106, 109), wherein the temporal spacing affects how frequent conductive communication messages are transmitted from the first device (102a, 102b, 104, 106, 109) to the second device (102a, 102b, 104, 106, 109), and the method further comprising:
after the changing to using the second timing scheme, monitoring a quality metric of the conductive communication with the second device when using the second timing scheme for performing the conductive communication with the second device (102a, 102b, 104, 106, 109);
detecting the quality metric being below the corresponding threshold, when using the second timing scheme for performing the conductive communication with the second device (102a, 102b, 104, 106, 109); and
in response to detecting the quality metric being below the corresponding threshold, when using the second timing scheme for performing the conductive communication with the second device (102a, 102b, 104, 106, 109), changing the temporal spacing.

13. The method of any one of claims 9 through 12, wherein:
the first device (102a, 102b, 104, 106, 109) includes and/or is communicatively coupled to at least three electrodes (108, 115, 125) and can perform the conductive communication using a plurality of different conductive communication vectors;
each of the plurality of different conductive communication vectors comprises a different combination of the at least three electrodes (108, 115, 125);
after the changing to using the second timing scheme for performing the conductive communication with the second device (102a, 102b, 104, 106, 109), monitoring a quality metric of the conductive communication with the second device (102a, 102b, 104, 106, 109), when the second timing scheme is being used to perform the conductive communication with the second device (102a, 102b, 104, 106, 109);
detecting the quality metric being below the corresponding threshold, when the second timing scheme is being used to perform the conductive communication with the second device (102a, 102b, 104, 106, 109); and
changing from using a first conductive communication vector of the plurality of different conductive communication vectors to using a second conductive communication vector of the plurality of different conductive communication vectors for performing the conductive communication with the second device (102a, 102b, 104, 106, 109), in response to the quality metric being below the corresponding threshold when using each of the first and second timing schemes to perform the conductive communication with the second device using the first conductive communication vector.

14. The method of any one of claims 9 through 13, wherein there is a temporal spacing between when one conductive communication message and a next conductive communication message is transmitted from the first device (102a, 102b, 104, 106, 109) to the second device (102a, 102b, 104, 106, 109), wherein the temporal spacing affects how frequent conductive communication messages are transmitted from the first device (102a, 102b, 104, 106, 109) to the second device (102a, 102b, 104, 106, 109), and the method further comprising:
after the changing to using the second conductive communication vector, monitoring a quality metric of the conductive communication with the second device (102a, 102b, 104, 106, 109) when using the second conductive communication vector for performing the conductive communication with the second device;
detecting the quality metric being below the corresponding threshold, when using the second conductive communication vector for performing the conductive communication with the second device (102a, 102b, 104, 106, 109); and
in response to detecting the quality metric being below the corresponding threshold, when using the second conductive communication vector for performing the conductive communication with the second device (102a, 102b, 104, 106, 109), changing the temporal spacing.

15. The method of any one of claims 9 through 14, wherein:
one of the first and second devices (102a, 102b, 104, 106, 109) comprises an IMD (102a, 102b, 104, 106) and the other one of the first and second devices (102a, 102b, 104, 106, 109) comprises an external device (109); or
each of the first and second devices (102a, 102b, 104, 106, 109) comprises a respective IMD (102a, 102b, 104, 106).
